# EUROPEAN PATENT APPLICATION

(11) **EP 4 738 381 A1**
(43) Date of publication of application: **06.05.2026**
(21) Application number: 25207933.0
(22) Date of filing: 10.10.2025
(51) Int. Cl.: G16H 40/20, G16H 50/00, G16H 30/40

(54) **MEDICAL IMAGING DEVICE PREDICTION AND RECOMMENDATION FOR OPTIMAL IMAGE QUALITY**

(30) Priority: 04.11.2024 US 202418935918
(71) Applicant: GE Precision Healthcare LLC, Waukesha, WI 53188 (US)
(72) Inventor: RODRIGUEZ, Ezra Nathaniel, Waukesha, WI 53188 (US); NOLAN, Jason, Fort Lauderdale, FL 33322 (US); HORBAIL, Suranjani, Naperville, IL 60563 (US); RANGAVAJHALA, Vamsee, Naperville, IL 60563 (US)
(74) Representative: Kilburn & Strode LLP

(57) **Abstract**

Techniques for predicting and recommending the best medical imaging device for fulling an order are presented. A system receives an order requesting performance of a medical imaging exam on a patient, the order comprising information identifying a modality, a clinical indication and an anatomical region for the exam. The system identifies different medical imaging devices capable of performing the exam based on the modality, and employs one or more first machine learning processes to estimate measures of quality of the exam capable of being generated by the different medical imaging devices based on the order information and historical information regarding historical medical imaging exams performed by the different medical imaging devices that satisfy the order information. The system selects a subset of the different medical imaging devices to recommend for fulfilling the order based on respective measures of quality for the subset satisfying a quality criterion.

## Description

### TECHNICAL FIELD

This disclosure relates generally to medical imaging, and more particularly to medical imaging device prediction and recommendation for optimal image quality (e.g., via employment of machine learning).

### BACKGROUND

Medical imaging devices of the same modality are not created equally. Within a single imaging modality (e.g., magnetic resonance imaging (MRI), computed tomography (CT), X-ray, and others), there are significant differences in terms of technology, performance, and capabilities. These variations can affect the quality of the images, the type of information captured, patient comfort and safety in terms of amount of radiation dosage exposure. Poor image quality can create a repeat exam scenario causing the patient to go back for the same imaging exam and may increase the radiation dosage given to the patient depending on the modality. Furthermore, the need for repeat imaging may cause a delay in treatment, especially if the patient is an outpatient. In addition, it is possible for exams to be unable to be performed at all if the device is not equipped to do so.

Accordingly, techniques for determining the best medical imaging device of amongst those of the same imaging modality available for fulfilling a medical imaging order that provides the best image quality while minimizing radiation exposure and the need for repeat imaging and while further accounting for a multitude of other scheduling considerations are desired.

### SUMMARY

The following presents a simplified summary of the specification in order to provide a basic understanding of some aspects of the specification. This summary is not an extensive overview of the specification. It is intended to neither identify key or critical elements of the specification, nor delineate any scope of the particular implementations of the specification or any scope of the claims. Its sole purpose is to present some concepts of the specification in a simplified form as a prelude to the more detailed description that is presented later.

According to an embodiment, a system includes at least one memory that stores computer-executable components, and at least one processor that executes the computer-executable components stored in the at least one memory. The computer-executable components can comprise a reception component that receives an order requesting performance of a medical imaging exam on a patient, the order comprising order information identifying a modality of the medical imaging exam, a clinical indication for the medical imaging exam, and an anatomical region to be captured in the medical imaging exam. The computer-executable components further comprise a filtering component that identifies different medical imaging devices capable of performing the medical imaging exam based on the modality, and an artificial intelligence (AI) component that employs more first machine learning processes to estimate measures of quality of the medical imaging exam capable of being generated by the different medical imaging devices based on the order information and historical imaging exam information regarding historical medical imaging exams performed by the different medical imaging devices that satisfy the order information. The computer-executable components further comprise an assessment component that selects a subset of the different medical imaging devices based on respective measures of quality for the subset satisfying a quality criterion, and a recommendation component that generates recommendation information identifying the subset and provides the recommendation information to at least one of, a device associated with the patient or a scheduling system configured to schedule the medical imaging exam for the patient in accordance with the recommendation information.

In one or more implementations, the computer-executable components further comprise a training component that trains one or more machine learning models to estimate training measures of quality corresponding to the measures of quality based on the historical imaging exam information, resulting in one or more trained versions of the one or more machine learning models, and an execution component that applies the order information and device information identifying the different medical imaging devices as input to the one or more trained versions to generate the measures of quality.

In some embodiments, the quality criterion comprises an acceptable measure of quality for the medical image exam. In an implementation of these embodiments, wherein the AI component further employs the one or more first machine learning processes or one or more second machine learning processes to estimate the acceptable measure of quality for the medical image exam based on the order information and the historical imaging exam information.

In some embodiments, the AI component further employs the one or more first machine learning processes or one or more second machine learning processes to estimate, based on the order information and the historical imaging exam information, radiation doses capable of being exposed to the patient via the different medical imaging devices in association with performing the medical image exam and achieving the acceptable measure of quality, wherein the assessment component selects the subset based on respective radiation doses for the subset satisfying a radiation dose criterion. In an implementation of these embodiments, the computer-executable components can further comprise a training component that trains one or more machine learning models to estimate training radiation doses corresponding to the radiation doses based on the historical imaging exam information, resulting in one or more trained versions of the one or more machine learning models, and an execution component that applies the order information, device information identifying the different medical imaging devices, and the acceptable measure of quality as input to the one or more trained versions to generate the radiation doses.

In some embodiments, elements described in connection with the disclosed systems can be embodied in different forms such as a computer-implemented method, a computer program product, or another form.

### BRIEF DESCRIPTION OF THE DRAWINGS

Numerous aspects, implementations, objects and advantages of the present invention will be apparent upon consideration of the following detailed description, taken in conjunction with the accompanying drawings, in which like reference characters refer to like parts throughout, and in which:
FIG. 1 illustrates a high-level block diagram of an example system that facilitates predicting and recommending the best medical imaging device(s) for fulfilling an imaging exam order, in accordance with one or more embodiments described herein;
FIG. 2 illustrates a high-level input/output (I/O) data flow associated with an order fulfillment evaluation component in accordance with one or more embodiments described herein;
FIG. 3 illustrates a high-level block diagram of an example order fulfillment evaluation component, in accordance with one or more embodiments described herein;
FIG. 4 illustrates an example machine learning process that facilitates predicting the best medical imaging device(s) for fulfilling an imaging exam order, in accordance with one or more embodiments described herein;
FIG. 5 illustrates an example training process for training one or more machine learning (ML) models to predict the best medical imaging device(s) for fulfilling an imaging exam order, in accordance with one or more embodiments described herein;
FIG. 6 illustrates an example computer-implemented method for predicting and recommending the best medical imaging device(s) for fulfilling an imaging exam order, in accordance with one or more embodiments described herein;
FIG. 7 illustrates another example computer-implemented method for predicting and recommending the best medical imaging device(s) for fulfilling an imaging exam order, in accordance with one or more embodiments described herein;
FIG. 8 illustrates another example computer-implemented method for predicting and recommending the best medical imaging device(s) for fulfilling an imaging exam order, in accordance with one or more embodiments described herein;
FIG. 9 illustrates another example computer-implemented method for predicting and recommending the best medical imaging device(s) for fulfilling an imaging exam order, in accordance with one or more embodiments described herein;
FIG. 10 illustrates an example computer-implemented method for updating one or more machine learning models to improve the accuracy of predictions related to the best medical imaging device(s) for fulling an exam imaging exam, in accordance with one or more embodiments described herein;
FIG. 11 is a schematic block diagram illustrating a suitable operating environment; and
FIG. 12 is a schematic block diagram of a sample-computing environment.

### DETAILED DESCRIPTION

The following detailed description is merely illustrative and is not intended to limit embodiments and/or application or uses of embodiments. Furthermore, there is no intention to be bound by any expressed or implied information presented in the preceding Background section, Summary section or in the Detailed Description section.

As described in the Background Section, medical imaging devices of the same modality are not created equally. Within a single imaging modality (e.g., MRI, CT, X-ray, and others), there are significant differences in terms of technology, performance, and capabilities. These variations can affect the quality of the images, the type of information captured, patient comfort and safety in terms of amount of radiation dosage exposure. Poor image quality can create a repeat exam scenario causing the patient to go back for the same imaging exam and may further increase the radiation dosage given to the patient and cause a delay in treatment.

With this context in mind, the disclosed subject matter is directed to systems, computer-implemented methods, apparatus and/or computer program products that facilitate predicting the best medical imaging device(s) of amongst those of the same imaging modality available for fulfilling a medical imaging order that provides the best image quality while minimizing radiation exposure and the need for repeat imaging, while further accounting for a multitude of other scheduling considerations. In this regard, the disclosed techniques go beyond finding a medical imaging device to schedule for an imaging exam that is capable of performing the exam in terms of modality and device capabilities, but further that can produce images with the quality needed to obviate duplicate imaging, therefore reducing potential over-irradiation dosage exposure to the patient while reducing patient imaging time overall.

To facilitate this end, the disclosed techniques employ artificial intelligence (AI) and machine learning (ML) techniques to learn correlations between a multitude of different factors represented in historical imaging exam data associated with the different medical imaging devices of the same modality that influence resulting imaging quality and radiation dosage, as tailored based on the clinical indication for the imaging exam, the anatomical region of interest (ROI) being imaged and different patient preferences and constraints (e.g., patient size, age, gender, comorbidities and conditions (e.g., pregnancy), implanted medical devices, and others). The disclosed techniques further employ AI and ML to learn requisite measures of quality needed for different types of imaging exams (e.g., in terms of modality, clinical indication, anatomical ROI, etc.) and patient considerations to ensure accurate diagnosis and treatment planning without repeat imaging. The requisite measure of quality can reflect an acceptable amount of noise (e.g., measured as a function of signal-to-noise ratio (SNR) or a similar metric) for respective images of the exam, an acceptable level of contrast (e.g., measured as a function of contrast-to-noise ratio (CNR) or a similar metric), an acceptable measure of temporal resolution, an acceptable measure of spatial resolution, an acceptable amount of artifacts (e.g., measured as a function of an amount and/or severity of artifacts such as blurring, streaks, and other types of artifacts) and/or other quality measures. In some implementations, the quality of respective images of an exam can include or correspond to a quality score or rating that accounts for one or more of these metrics and/or a subjective interpretation of the images as provided by a reviewing entity (e.g., a radiologist or the like) and/or an image quality scoring algorithm.

The disclosed techniques further employ AI and ML to learn possible acquisition protocols and parameters that can be employed by different imaging devices of the same modality that can be used to achieve the requisite measures of quality and how the different acquisition protocols/parameters influence the amount of radiation exposed to the patient along with imaging exam duration.

Based on the above-described information learned from the historical data, the disclosed subject matter can train one or more ML models to predict the optimal imaging device(s) for fulfilling an imaging exam order for a patient that maximizes quality while minimizing radiation exposure and the need for repeat imaging while also accounting for patient preferences and constraints. For example, in some embodiments, the disclosed techniques can be implemented in the form of a system that receives medical imaging orders and generates recommendation information for the respective orders identifying the optimal imaging device or devices for fulfilling for the respective orders. In some embodiments, the system can provide the recommendation information to the patient (i.e., via. an electronic device associated with the patient, such as a smartphone, a computer or the like) to enable the patient to select and schedule the imaging exam with an optimal imaging device. Additionally, or alternatively, the system can provide the recommendation information to a scheduling system (or scheduling component included with the system) that employs the recommendation information to automatically schedule the imaging exam for the patient with an optimal imaging device.

The disclosed system can further regularly update or tune the one or more ML models over time to improve the accuracy of the optimal device predications based on tracking and evaluating information post recommendations regarding whether and to what extent respective imaging exams performed in accordance with the recommendation information achieved the expected image quality, radiation exposure, and eliminated or reduced the need for repeat imaging.

Compared to a medical image exam scheduling system that assumes all imaging devices of the same modality are created equal, the disclosed system employs AI and ML to predict the best medical imaging device(s) of amongst those of the same imaging modality available for fulfilling a medical imaging order that can provide the best or requisite image quality while minimizing radiation exposure and the need for repeat imaging. As such, the disclosed system can reduce or eliminate costs associated with repeat imaging, including clinical and financial costs attributed to delays in accurate diagnosis and treatment planning, thus improving clinical outcomes and saving lives. Furthermore, technical advantages of the systems described herein can enable reducing memory storage used for capturing medical images and reduced time for processing medical images by reducing or eliminating repeat imaging.

As used herein, the term medical imaging device refers to a machine used in healthcare to create visual representations (i.e., medical images) of the inside of a body. Various different types of medical imaging devices exist that vary with respect to modality, that is the specific type of imaging technique or method used to create images of the body. Common types of medical imaging devices include, but are not limited to, X-ray devices, digital radiography (DX) X-ray devices, X-ray angiography (XA) devices, panoramic X-ray (PX) images, computerized tomography (CT) device, mammography (MG) devices (including tomosynthesis devices), magnetic resonance imaging (MRI) device, ultrasound (US) device, color flow doppler (CD) devices, position emission tomography (PET) devices, single-photon emissions computed tomography (SPECT) device, nuclear medicine (NM) devices, and the like. Most medical imaging devices include or incorporate computer processing technology for several critical functions, ranging from image acquisition to advanced processing and analysis. To this end, the term medical image device refers to the entirety of the machine or machines (e.g., including one or more computers and associated processing technology/software) used by the medical image device to acquire raw image data and convert the raw image data into usable medical images.

The terms "medical image," "medical image data," and the like are used to refer to image data that depicts one or more anatomical regions of a patient. Reference to a medical image or medical image data herein can include any type of medical image associated with various types of medical image acquisition/capture modalities. For example, medical images can include (but are not limited to): radiation therapy (RT) images, XR images, DX-ray images, XA images, PX images, CT images, MG images, MRI images, US images, CD images, PET images, SPECT images, NM images, and the like. Medical images can also include synthetic versions of native medical images such as augmented, modified or enhanced versions of native medical images, augmented versions of native medical images, and the like generated using one or more image processing techniques. In some embodiments, the term "image data" can include the raw measurement data (or simulated measurement data) used to generate a medical image (e.g., the raw measurement data captured via the medical image acquisition process).

The terms "algorithm" and "model" are used herein interchangeably unless context warrants particular distinction amongst the terms. The terms "artificial intelligence (AI) model" and "machine learning (ML) model" are used herein interchangeably unless context warrants particular distinction amongst the terms. Reference to an AI or ML model herein can include any type of AI or ML model, including (but not limited to): deep learning (DL) models, neural network models, deep neural network models (DNNs), convolutional neural network models (CNNs), generative adversarial neural network models (GANs), transformer models, and the like. An AI or ML model can include supervised learning models, unsupervised learning models, semi-supervised learning models, combinations thereof, and models employing other types of ML learning techniques. An AI or ML model can include a single model or a group of two or more models (e.g., an ensemble model, chained models, or the like).

One or more embodiments are now described with reference to the drawings, wherein like referenced numerals are used to refer to like elements throughout. In the following description, for purposes of explanation, numerous specific details are set forth in order to provide a more thorough understanding of the one or more embodiments. It is evident, however, in various cases, that the one or more embodiments can be practiced without these specific details.

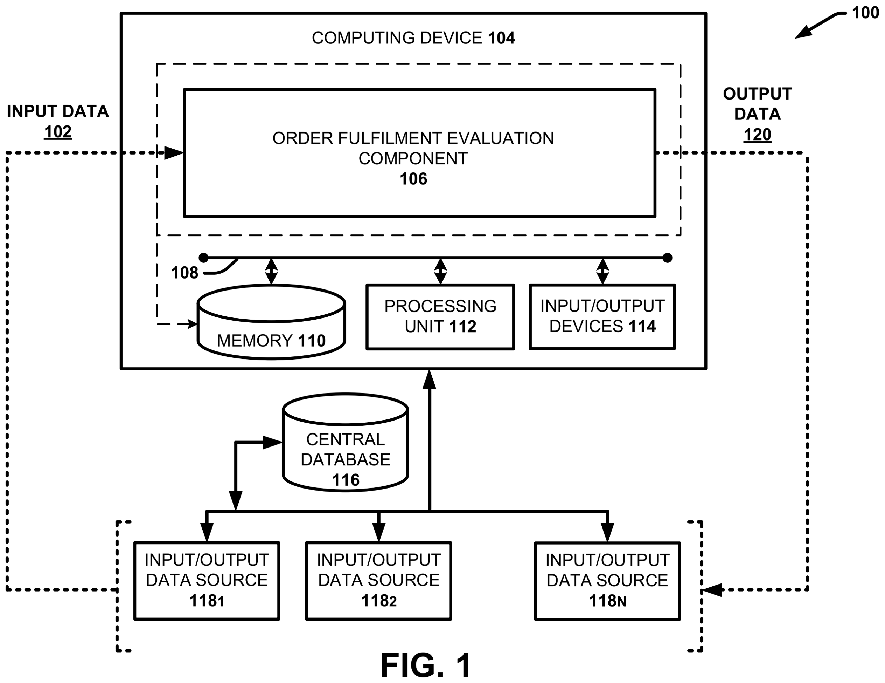

Turning now to the drawings, FIG. 1 illustrates a high-level block diagram of an example system 100 that facilitates predicting and recommending the best medical imaging device(s) for fulfilling an imaging exam order, in accordance with one or more embodiments described herein. System 100 can include or correspond to one or more computing devices, machines, virtual machines, computer-executable components, datastores, and the like that may communicatively coupled to one another either directly or via one or more wired or wireless communication frameworks. Aspects of the systems, apparatuses or processes explained in this disclosure can constitute computer-executable or machine-executable component(s) embodied within machine(s), e.g., embodied in one or more computer readable mediums (or media) associated with one or more machines. Such component(s), when executed by the one or more machines, e.g., computer(s), computing device(s), virtual machine(s), etc. can cause the machine(s) to perform the operations described.

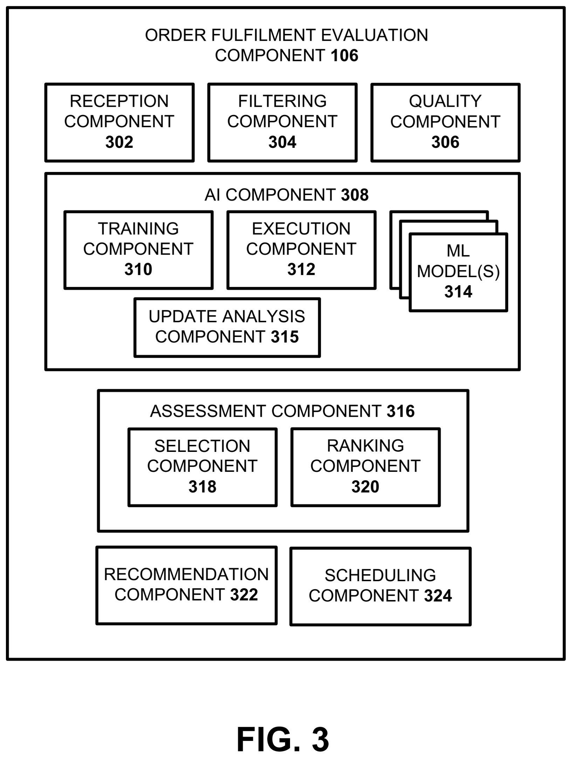

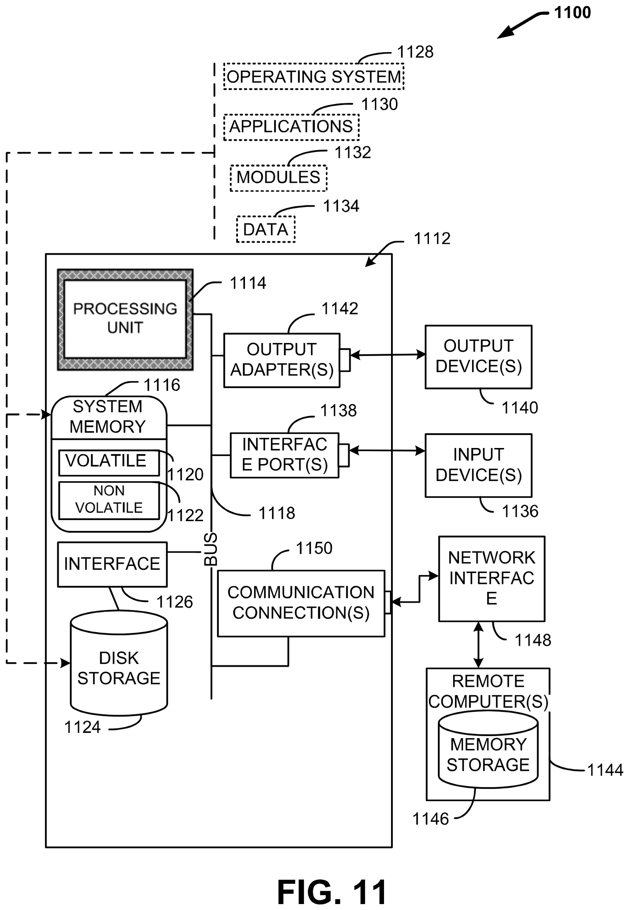

In this regard, system 100 can include at least one computing device 104 that comprises at least one memory 110 that stores computer-executable components, and at least one processor or processing unit 112 that executes the computer-executable components stored in the at least one memory 110. The computer-executable components comprise order fulfillment evaluation component 106 and associated components illustrated in FIG. 3. Examples of said memory 110 and processing unit 112 as well as other suitable computer or computing-based elements, can be found with reference to FIG. 11 (e.g., system memory 1106 and processing unit 1114 respectively), and can be used in connection with implementing one or more the components shown and described in connection with FIG. 1, or other figures disclosed herein.

Computing device 104 can further include one or more input/output devices 114 to facilitate receiving user input and rendering data (e.g., output data 120) to users in association with performing various operations described with respect to the order fulfilment evaluation component 106 and/or processes described herein. Suitable examples of the input/output devices 114 are described with reference to FIG. 11 (e.g., input devices 1136 and output devices 1140). Computing device 104 can further include a system bus 108 that couples the memory 110, the processing unit 112 and the input/output devices 114 to one another.

Computing device 104 can be implemented on or in connection with a network of systems, devices, and databases associated with an enterprise application (e.g., an enterprise network of connected machines) that provides input data 102 to be processed by the order fulfilment evaluation component 106 and/or receives output data 120 generated by the order fulfilment evaluation component. For example, computing device 104 can be connected to various input/output data sources 118_{1-N} (e.g., a radiology information system (RIS), a medical image exam ordering system, medical image storage and archiving systems, medical imaging devices themselves or provider systems associated with the respective medical imaging devices, one or more electronic health record (EHR) and/or electronic medical record (EMR) databases, medical imaging exam scheduling systems, patient devices, patient medical management systems, one or more clinical information databases/systems, one or more clinical domain knowledge databases etc.), and/or a central database 116 that provides the input data 102 for processing by the order fulfilment evaluation component 106 and/or receives the output data 120 generated by the order fulfilment evaluation component 106.

Generally, the order fulfilment evaluation component 106 facilitates receiving input data 102 comprising orders for medical imaging exams to be scheduled for patients and employs AI and/or ML techniques to generate output data 120 that identifies or indicates the best medical imaging device(s) available to the patients for fulfilling the imaging exam orders. To this end, assuming the order specifies a modality required for the imaging exam, a clinical indication for the exam, and an anatomical ROI to be captured via the exam, reference to the "best" medical device or devices for fulfilling an order as used herein refers to a select medical imaging device (or a select subset of medical imaging devices, the subset comprising one or more devices) included within a group of medical imaging devices of the same modality that are available to the patient (e.g., based on a geographical location of the patient, insurance coverage accepted, and optionally scheduling availability given an urgency level for performance of the exam, and potentially other factors) that can generate medical images of sufficient quality needed to ensure accurate diagnosis without requiring repeat imaging (or minimizing the possible need for repeat imaging). The "best" medical imaging device or devices can also account for the best device or devices within the same modality available to the patient that can provide the sufficient quality while also minimizing the amount of radiation dose exposed to the patient.

In this regard, different modalities are better suited for visualizing specific tissues (e.g., bones, soft tissues, blood flow) or functions (e.g., metabolic activity, motion). Each modality varies in terms of detail, radiation exposure, and clinical application. The choice of modality depends on the clinical question, the part of the body being examined, and the type of pathology suspected. Imaging exam orders are prescribed by an ordering physician after evaluating a patient. The imaging exam order typically includes information identifying the patient (e.g., via a unique identifier (ID) such as the patient's name, or another type of anonymous ID), the ordering physician, the clinical indication for the exam or exam (e.g., a detailed explanation of why the imaging is being ordered, such as the known or suspected diagnosis, symptoms, clinical findings, etc.), the specific modality of the exam, and the ROI to be imaged (e.g., the specific body part or anatomical region to be imaged). In some cases, the order may also include additional information, such as special instructions (e.g., any particular imaging techniques or acquisition protocols/required, such as with or without contrast, high-resolution imaging, etc.), a level of urgency of the exam, and relevant patient preference and/or constraints associated with performance of the exam (e.g., scheduling preferences, patient physiological constraints (e.g., regarding IMDs, pregnancy, patient demographics (e.g., height, weight, body size, age, and other important parameters for determining the appropriate imaging device and acquisition protocols/parameters).

Depending on the usage scenario and the healthcare environment, the ordering physician may provide the patient with a paper form of the order and direct the patient to personally select and schedule the exam at any medical imaging device provider available to the patient (e.g., based on the patient's location, insurance coverage, etc.) that provides an imaging device of the modality prescribed for the exam. In other scenarios, the imaging order may be electronically entered into a radiology information system (RIS) that facilitates scheduling the exam for the patient with any medical imaging device provider available to the patient that provides an imaging device of the modality prescribed for the exam.

However, medical imaging devices of the same modality can vary significantly based on several factors, including their design, technical capabilities, features, and intended clinical applications. Even within the same modality, such as X-ray, CT, MRI, and others, different devices are optimized for specific tasks, patient populations, or imaging environments. For example, different medical imaging devices within the same modality can vary with respect to resolution and image quality capabilities, field strength capabilities (for MRI), detector technology (for X-ray and CT) capabilities, slice thickness capabilities (for CT and MRI), speed and throughput, size and portability, field of view (FOV), contrast capabilities, software and post-processing capabilities, radiation dose exposure (for X-ray, CT and nuclear medicine), cost and accessibility.

More particularly, within the same modality, some medical imaging devices are optimized for higher spatial resolution, which allows for finer detail. For example, an X-ray machine used for mammography typically provides much higher resolution compared to a general-purpose chest X-ray machine. In modalities like MRI or ultrasound, some devices are designed to capture images faster (higher temporal resolution) for dynamic studies, such as heart motion, while others prioritize high spatial resolution for still images, like brain scans. MRI scanners can vary based on the strength of their magnetic fields which can impact their suitability for different clinical indications. For example, low-field MRI (0.2T-1.5T) is sufficient for routine brain or joint imaging, while high-field MRI (3T or higher) provides better signal-to-noise ratio and more detailed images, especially for tasks like neuroimaging or detecting subtle tissue changes. MRI scanners also include ultra-high field MRI (7T) scanners, which are specialized systems used in research or advanced clinical settings, offering extremely high resolution but often at the expense of longer scan times and increased susceptibility to artifacts.

The detector technology can also vary amongst medical imaging devices of the same modality (e.g., for X-ray, CT, etc.). For example, older X-ray systems use film-based or analog detectors, while modern X-ray systems use digital detectors, which offer better image quality, faster acquisition times, and improved workflow. Some digital X-ray systems use direct conversion detectors (better spatial resolution), while others use indirect detectors, which are generally more affordable but may have lower image resolution.

CT and MRI machines also vary with respect to slice thickness capabilities, that is the thickness of respective volume regions covering the ROI of the patient captured in a series of images acquired for the exam. In this regard, the number of slices and the thickness of each slice can vary across different CT and MRI scanners. Modern multi-slice CT scanners (e.g., 64-slice or 128-slice CT) can capture thinner slices (sub-millimeter) and larger volumes quickly, which is critical for detailed anatomical imaging and reducing motion artifacts. Older scanners with fewer slices (e.g., 4- or 16-slice CT) may produce thicker slices, resulting in less detailed images. Similarly, MRI devices can offer different slice thickness options, with higher-end systems allowing ultra-thin slices for more precise imaging, particularly in areas like the brain or joints.

Imaging devices of the same modality can also vary with respect to speed and throughput capabilities. For example, some devices are designed to capture images quickly, which is important for critically ill patients or imaging in emergency settings (e.g., a CT scanner in a trauma center). Others prioritize higher resolution and detail, which may take longer to acquire (e.g., MRI for neuroimaging).

Imaging devices of the same modality can also vary with respect to size and portability. In many modalities, devices can be either stationary or portable. For example, portable X-ray machines can be wheeled to a patient's bedside in an ICU or trauma unit, whereas larger, stationary X-ray systems are used in dedicated imaging rooms. Similarly, ultrasound systems range from large, cart-based machines to compact, hand-held units designed for bedside use or point-of-care imaging in emergency situations.

Imaging devices of the same modality can also vary with respect to FOV capabilities and accommodating patients of varying sizes. For example, MRI systems vary in the size of their bore (the tunnel through which the patient enters). A wide-bore MRI has a larger diameter, which can accommodate larger patients or help reduce claustrophobia but may have slightly different image quality compared to standard-bore MRI systems.

In addition, some medical imaging devices within the same modality are optimized for specific body parts and clinical indications. For instance, dedicated extremity MRI systems are designed specifically for scanning limbs (hands, wrists, knees), offering a smaller, more comfortable experience for the patient and often more focused, higher-resolution images than a full-body MRI. Some CT scanners are optimized for cardiac imaging, capable of capturing high-resolution images of the coronary arteries in a single heartbeat, while others may be more general-purpose. Certain MRI systems are designed for use in an operating room during surgeries, allowing for real-time imaging during a procedure, which differs from standard MRI systems used for diagnostic purposes.

The ability to administer and process contrast-enhanced exams can also vary between devices of the same modality. For example, some devices are designed to work with contrast agents to improve visibility of certain tissues or structures while other are not. For instances, CT scanners that perform CT angiography use iodine-based contrast to visualize blood vessels, while MRI scanners use gadolinium-based contrast to enhance soft tissues. In addition, some CT scanners are equipped with dual-energy capabilities, allowing them to capture images at two different energy levels. This provides enhanced contrast differentiation between tissues and materials like bone, fat, and soft tissue.

Medical imaging devices of the same modality can also vary in terms of the software they use for image acquisition and processing. Higher-end systems may include advanced algorithms for 3D reconstruction, image fusion (combining images from different modalities like PET/CT), or automated segmentation of organs or tumors. In addition, some newer systems come equipped with AI tools that assist with image interpretation, image quality enhancement, and automate tasks like lesion detection, or provide decision support, which might not be available on older or more basic models.

Further, in CT and X-ray modalities the radiation dose exposure ranges to the patient can vary for different machines of the same modality. For example, newer systems are designed to minimize radiation exposure to patients while maintaining image quality. Low-dose CT scanners are particularly important for pediatric imaging or repeated scans, such as in cancer follow-up. Modern devices include dose monitoring features that help radiologists and technicians optimize the radiation dose used during each scan, providing real-time feedback on radiation safety.

Furthermore, the operating technician of the imaging exam plays a role in the quality of the resulting medical images by influencing several factors throughout the imaging process. The technician's expertise and attention to detail play a critical role in the outcome of a medical imaging exam. From patient positioning and choosing appropriate settings to managing motion and ensuring safety, their actions directly impact the quality and diagnostic value of the resulting images. Skilled technicians ensure that images are clear, properly exposed, and free from artifacts, allowing radiologists and clinicians to make accurate diagnoses. Different technicians operating the same type of imaging device (e.g., in terms of modality) can thus have different skill sets tailored to different types of patients, ROIs, and clinical indications/applications or the exam, which can influence the quality and diagnostic value of the resulting images as well as the amount of radiation exposed to the patient during the exam.

With this context in mind, the order fulfilment evaluation component 106 employs AI and ML techniques to learn correlations between a multitude of different factors represented in historical imaging exam data associated with the different medical imaging devices of the same modality that influence resulting imaging quality and radiation dosage, as tailored based on the clinical indication for the imaging exam, the anatomical ROI being imaged and different patient preferences and constraints (e.g., patient size, age, gender, comorbidities and conditions (e.g., pregnancy), implanted medical devices (IMDs), and others). In various embodiments, image quality can be measured as a function of an amount of average amount of noise (e.g., an SNR metric or a similar metric) included in collective images of an exam, a level of contrast (e.g., measured as a function of CNR or a similar metric), a measure of temporal resolution, a measure of spatial resolution, a measure of an amount and/or severity of artifacts (e.g., blurring, streaks, and other types of artifacts) and/or other quality measures. In some implementations, the quality of respective images of an exam can include or correspond to a quality score or rating that accounts for one or more of these metrics and/or a subjective interpretation of the images as provided by a reviewing entity (e.g., a radiologist or the like) and/or an image quality scoring algorithm.

The order fulfilment evaluation component 106 can also employ AI ad ML techniques to learn requisite measures of quality needed for different types of imaging exams (e.g., in terms of modality, clinical indication, anatomical ROI, etc.) and patient considerations (e.g., patient size, height, weight, age, gender, comorbidities, preferences, urgency need of the exam, etc.) to ensure accurate diagnosis and treatment planning without repeat imaging. The requisite measure of quality can reflect an acceptable amount of noise (e.g., measured as a function of SNR or a similar metric) for respective images of the exam, an acceptable level of contrast (e.g., measured as a function CNR or a similar metric), an acceptable measure of temporal resolution, an acceptable measure of spatial resolution, an acceptable amount of artifacts (e.g., measured as a function of an amount and/or severity of artifacts such as blurring, streaks, and other types of artifacts) and/or other quality measures, such as an acceptable quality score that accounts for one or more of these quality measures. The order fulfilment evaluation component 106 can also employ AI and ML to learn possible acquisition protocols and parameters that can be employed by different imaging devices of the same modality that can be used to achieve the requisite measures of quality and how the different acquisition protocols/parameters influence the amount of radiation exposed to the patient along with imaging exam duration. Based on the above-described information learned from the historical data, the order fulfilment evaluation component can predict the optimal imaging device(s) for fulfilling new imaging exam orders for patients that maximize quality while minimizing radiation exposure and the need for repeat imaging while also accounting for patient preferences and constraints.

To facilitate this end, the input data 102 can comprise a plethora of relevant information regarding details of the orders, the patients for which the orders are associated and historical exam information regarding past imaging exams performed for historical patients.

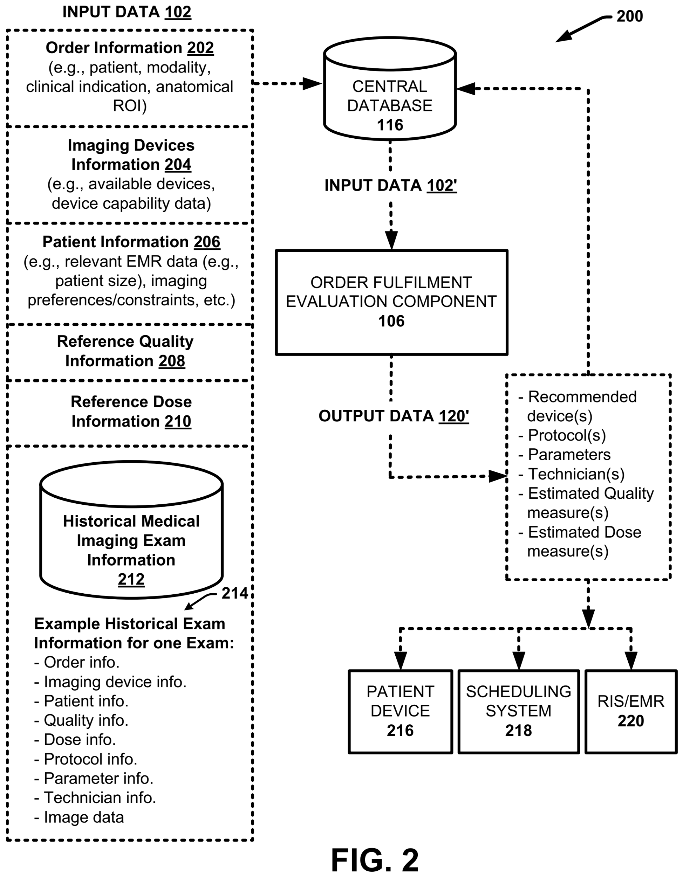

In this regard, FIG. 2 illustrates a high-level input/output (I/O) data flow 200 associated with the order fulfillment evaluation component 102 in accordance with one or more embodiments described herein. With reference to FIGs. 1 and 2, in various embodiments, the input data 102 can include, (but is not limited to), order information 202, imaging devices information 204, patient information 206, reference quality information 208, reference dose information 210 and historical medical imaging exam information 212.

The order information 202 can include or correspond to information included with or associated with respective medical image exam orders prescribed for patients. The order information 202 can represent a wide range of different imaging exam orders to be scheduled for different patients. For example, in some embodiments, the imaging exam orders (comprising the order information 202 for respective patients/orders) can be entered electronically into a RIS (e.g., an input/output data source of the input/output data sources 118_{1-N} corresponding to a RIS) coupled to the computing device 104. The order fulfillment component 106 can further monitor the RIS and evaluate new orders as they are entered/received at the RIS.

A radiology information system (RIS) is a specialized healthcare system used in radiology departments to manage medical imaging records and related data. It plays a crucial role in the workflow of radiology departments, enabling the management of patient data, imaging procedures, results, and administrative tasks. In some embodiments, the RIS can be integrated with picture archiving and Communication Systems (PACS) and/or EMRs (also included in the input/output data sources 118_{1 -N}) to ensure seamless access to patient information and medical images across healthcare providers. For example, in some embodiments, an integrated RIS/EMR 220 can serve as both an input data 102 source and an output data 120 source, such that recommendation data regarding the "best" device(s) for fulfilling respective orders can be sent back to the RIS/EMR 220 and associated with the patient's medical record and employed for scheduling the exam (e.g., via the RIS and/or another dedicated scheduling system 218 coupled to the RIS/EMR and the computing device 104). The RIS typically handles the administrative and operational aspects of radiology services, while PACS is responsible for storing, retrieving, and viewing medical images. Together, RIS and PACS form the backbone of radiology departments, streamlining both image management and workflow processes.

In this regard, the order information for individual orders can include but is not limited to, information identifying the patient (e.g., via a unique patient ID), the modality, the clinical indication for the exam, and the anatomical ROI to be captured in the exam. In some implementations, the order information may also include additional details describing relevant attributes of the patient for the exam, such as but not limited to, the patient's size (e.g., height, weight), age, gender, comorbidities, patient preferences (e.g., regarding imaging), and patient constraints for the imaging exam (e.g., comorbidity based constraints, urgency constraints, and others). Additionally, or alternatively, such (relevant) patient information 206 can be included in or otherwise provided by a separate data source (e.g., pain an electronic medical record (EMR) for the patient that can be accessed and extracted by the order fulfilment component 106 using the unique ID for the patient. For example, the patient information 206 can include EMR data for respective patients for which orders are received (and historical orders as included in the historical medical exam information 212). The EMR data can include a wealth of information about respective patient's medical records, demographic information, patient imaging exam preferences and constraints and the like. In some embodiments, the order information 202 may also include special instructions for the exam, such as desired acquisition protocols/parameters (e.g., contrast vs. non-contrast, high-resolution imaging, low resolution imaging, etc.) an urgency level of the exam, and the like.

The imaging devices information 204 can include or correspond to information identifying available imaging devices that can be used to fulfil the orders. For example, the imaging devices information 204 can identify all available imaging devices within a defined geographical area (e.g., a town, a city, a state, etc.) or the like. Reference to an "available" imaging device means that the imaging device exists and is accessible to a patient for performing the exam prescribed in an order (e.g., in terms of location, modality required and optionally insurance coverage accepted). In this regard, an imaging device is considered available regardless as to its current scheduling availability for scheduling a particular exam. As described in greater detail below, in some embodiments, after the order fulfillment evaluation component 106 has predicted the best device or devices for fulfilling an order, a scheduling system 218 (and/or the RIS) can optionally further determine and recommend available scheduling timeslot of the best device or devices to schedule the order. In some implementations, this can involve receiving a ranked list of two or more best imaging devices and determining the ultimate device for scheduling the exam from the ranked list based on the timeslot availability of the respective ranked devices and the urgency level of the exam (and potentially other patient scheduling preferences and other factors (e.g., the availability of a particular technician to perform the exam, wherein the particular best imaging devices were selected under the constraint that the exam is to be performed by the particular technician.).

The imaging devices information 204 can also include device capability data describing the capabilities of the respective imaging devices represented in the imaging devices information 204. In this regard, the device capability data can include information identifying the modality of each imaging device, acquisition protocols/parameters that can be employed by the respective devices, and various other detailed information regarding specific capabilities of the respective devices as tailored based on modality (e.g., spatial resolution capabilities, temporal resolution capabilities, field strength capabilities (for MRI), detector technology (for MRI, CT, X-ray, etc.) slice thickness capabilities (for CT and MRI), speed and throughput capabilities, size and portability capabilities, FOV capabilities, contrast capabilities, software and post-processing capabilities, radiation dose profiles, and specific clinical application capabilities (e.g., tailored to specific ROIs and/or clinical indications, such as CT scanners specialized for high-resolution cardiac imaging and the like). In some embodiments, the imaging devices information 204 can also identify acquisition protocols and acquisition parameters that can be configured for the respective imaging devices. In some embodiments, the imaging devices information 204 can also include information identifying current technicians performing respective types of imaging exams using the corresponding imaging devices. In some embodiments, the imaging devices information 204 can be provided by the RIS, or another system or database of the input/output data sources 118_{1-N}.

The reference quality information 208 can include or correspond to information that defines preferred or acceptable (i.e., requisite) quality metrics of medical images obtained for different types of imaging exams in terms of modality, ROI and clinical indication for the exam. The reference quality metrics can include objective metrics for different exam types, regarding acceptable measures of SNR, CNR, spatial resolution, temporal resolution, artifacts and other quality measures, such as an acceptable quality score that accounts for a combination of different quality metrics. In some embodiments, reference quality information 208 and be provided by the RIS, or another system or database of the input/output data sources 118_{1-N}. In some embodiments, the order fulfilment evaluation component 106 can determine and define the reference quality information and store the reference quality information in local memory of the computing device (e.g., memory 110) or another information storage system accessible to the order fulfilment evaluation component 106.

Similarly, the reference dose information 210 can include or information that defines preferred radiation dosage levels for different types of imaging exams in terms of modality, ROI and clinical indication for the exam. For example, in some implementations, the reference dose information 210 can include diagnostic reference levels (DRLs) for the different types of exams. DRLs are used to promote good practice in minimizing radiation exposure while still obtaining clinically adequate images. For each type of exam, DRLs represent dose values based on national or regional data collected from many hospitals or clinics. These values are typically set at the 75th percentile of dose distributions for specific procedures, meaning most exams should use doses below the DRL. In some embodiments, reference quality information 208 and be provided by the RIS, or another system or database of the input/output data sources 118_{1-N}., such as a clinical information system/database providing domain knowledge or the like.

Patient factors also play a significant role in determining the acceptable radiation dose for medical imaging procedures that use ionizing radiation, such as X-rays, CT scans, and nuclear medicine. These factors include the patient's age, body size, health condition. Tailoring the radiation dose to the individual patient is important to ensure diagnostic accuracy while minimizing unnecessary exposure to radiation risks. In some embodiments, within each exam type, the reference quality information 208 can also provide tailored reference dose levels for different patient profiles accounting for different patient factors, including age, body size, and health status.

For example, children are more sensitive to radiation because their cells are dividing more rapidly, making them more susceptible to radiation-induced DNA damage. Moreover, they have a longer expected lifespan, increasing the time for radiation-induced effects (like cancer) to manifest. Due to these risks, pediatric imaging protocols are adjusted to use the lowest possible radiation dose that still provides sufficient image quality. While older adults are less susceptible to the long-term risks of radiation-induced cancer due to their shorter remaining lifespan, they may have age-related health conditions that necessitate careful dose management. In addition, many elderly patients have comorbidities (e.g., cardiovascular disease, osteoporosis) that require frequent imaging procedures. Cumulative radiation exposure over multiple studies may increase their risk of adverse effects, so dose management remains important. Body size and composition also influence acceptable radiation doses for imaging exams. In larger patients, more radiation is often needed to penetrate the body and produce a clear image. Depending on the exam type, higher doses for obese patients are often necessary to avoid grainy or poor-quality images that could miss critical diagnostic details. However, efforts are made to minimize the dose by optimizing settings such as adaptive dose modulation (which adjusts the dose in real-time based on body part thickness). On the other hand, smaller or leaner patients generally require lower doses because there is less tissue for the radiation to penetrate, and lower photon counts can still produce clear images. Overexposure in these patients can result in unnecessary radiation doses.

Appropriate dose levels for different imaging exam types can also vary based on gender. For example, females are generally more sensitive to radiation-induced cancer, particularly breast and thyroid cancers. As a result, extra precautions may be taken to minimize radiation dose in female patients, especially when imaging these sensitive areas. Pregnancy status of females can also influence the amount of acceptable radiation dose. The developing fetus is highly sensitive to radiation, especially during the first trimester, when organs and tissues are forming. Even small doses of ionizing radiation can increase the risk of developmental abnormalities, miscarriage, or childhood cancer. For pregnant women, alternative imaging modalities that do not use ionizing radiation (such as ultrasound or MRI) are preferred whenever possible. If a radiation-based scan is necessary (e.g., in emergencies), protocols are adjusted to use the lowest possible dose, and lead shielding is used to protect the fetus.

The reference dose levels for different types of imaging exams can also account for other comorbidities and preexisting conditions. Some patients may have genetic conditions, such as Li-Fraumeni syndrome, which predispose them to a higher risk of cancer, including radiation-induced cancer. In these cases, even more stringent measures are taken to minimize radiation exposure. Patients with compromised immune systems (e.g., due to chemotherapy or autoimmune diseases) may be more vulnerable to the harmful effects of radiation, necessitating lower-dose imaging protocols.

The historical medical imaging exam information 212 can include information regarding past exams performed via the respective available imaging devices represented in the imaging devices information 204, including details of the past exams, the patients involved and the results of the exams (e.g., actual medical images, radiology reports, and historical imaging history data for respective patients indicating whether the exam required one or more repeat exams, and the like). The past exams can cover all types of imaging exams performed by the respective imaging devices in terms of modality, ROI and clinical indication. In an example 214, the historical medical imaging exam information 212 for one exam can include the order information (corresponding to the order information 202 described above for an individual order), the imaging device used, relevant information about the patient (e.g., size, height, weight, age, gender, comorbidities, patient preferences and patient constraints), information describing the resulting quality of the exam (e.g., resulting image resolution (e.g., temporal and spatial), SNR metric(s), CNR metric(s), contrast metric(s), artifacts (e.g., information describing measures severity and/or amount of artifacts such as blurring, streaks, and other artifacts, etc.), and one or more subjective quality measures (e.g., such as a quality score provided by the reviewing physician/radiologist indicating whether and to what degree the quality of the images was diagnostically acceptable or not), radiation dose information identifying or indicating the radiation dose exposed to the patient, acquisition protocol information describing the acquisition protocols used, acquisition parameter information describing the acquisition parameters used, technician information identifying the technician that performed the exam, and optionally the actual image data obtained for the exam. The historical imaging exam information 212 for respective past exams can also include information describing the results of the exam, such as the actual clinical findings such as the observations realized in the image data and details of the observations (e.g., size, location, morphology of lesions/observations and the like).

In some embodiments, various components of the historical imaging exam information 212 for a past exam may be included with the actual medical image data (e.g., as provided in structured reports associated therewith, as included in metadata associated therewith, or the like) in accordance with the digital imaging and communication in medicine (DICOM) or the like. For example, the historical medical imaging exam information 212 can include DICOM files for the past exams. DICOM files typically store medical images from modalities like X-ray, CT, MRI, Ultrasound, PET, and more. The image data is stored in a format that maintains the full fidelity of the original scan, ensuring that the diagnostic quality is preserved. Each DICOM file contains not just the image, but also metadata that describes the image and provides critical information related to the scan. This metadata can include the patient information (e.g., name, ID, date of birth, gender, etc.), imaging details (e.g., modality, image resolution, slice thickness, date and time of the scan, etc.), imaging device information (e.g., details about the specific imaging device used), and exam and series information (e.g., the type of exam, body part or ROI being imaged, and acquisition parameters and protocols used).

As illustrated in FIG. 2, in some embodiments, some or all of the input data 102 can be aggregated into a central database 116 accessible to the order fulfilment evaluation component 106 as received from respective sources (e.g., input/output data sources 118_{1-N}) of the input data 102 (e.g., in real-time or as needed for ML training and/or new order processing by the order fulfilment evaluation component 106). In this regard, the input/output data sources 118_{1-N} can include or correspond to various disparate medical systems, medical information systems, medical devices, patient devices, and/or databases that generate, store or otherwise provide the input data 102. For example, the input/output data sources 118_{1-N} can include but are not limited to a RIS, a RIS/EMR a medical image exam ordering system, medical image storage and archiving systems (e.g., a PACS or the like), medical imaging devices themselves or provider systems associated with the respective medical imaging devices, EHR/EMR databases/systems, medical imaging exam scheduling systems, patient devices, patient medical management systems, one or more clinical information databases/systems, one or more clinical domain knowledge databases etc.

As noted above, the order fulfilment evaluation component 106 employs AI and ML techniques to learn correlations between a multitude of different factors represented in the historical imaging exam information 212 that influence resulting imaging quality and radiation dosage for different types of imaging exams (e.g., in terms of modality, clinical indication and ROI) performed by the respective imaging devices represented in the imaging devices information 204 and different patients (e.g., accounting for different patient demographics, preferences and constraints). The order fulfilment evaluation component 106 can also employ AI and ML techniques to learn (e.g., based on the historical imaging exam information 212 and in some implementations the reference quality information 208), requisite measures of quality needed for the different types of imaging exams (e.g., in terms of modality, clinical indication, anatomical ROI, etc.) and the different patients to ensure accurate diagnosis and treatment planning without repeat imaging. The order fulfilment evaluation component 106 can also employ AI and ML to learn (e.g., based on the historical imaging exam information 212, the imaging devices information 204 and the reference dose information 210) possible acquisition protocols and parameters that can be employed by different imaging devices of the same modality that can be used to achieve the requisite measures of quality and how the different acquisition protocols/parameters influence the amount of radiation exposed to the patient along with imaging exam duration.

Based on the above-described information learned from the historical data, the order fulfillment evaluation component 106 can evaluate/analyze input data 102' for a new order and predict the optimal imaging device(s) for fulfilling the new order that maximizes quality while minimizing radiation exposure and the need for repeat imaging while also accounting for patient preferences and constraints. For example, the output data 120' for a new order generated by the order fulfillment evaluation component 106 can include (but is not limited to) one or more recommended devices for fulfilling the order, recommended acquisition protocols, recommended acquisition parameters, recommended technicians, estimated quality measures to be obtained in the resulting images and estimated dose measures to be obtained in the resulting images. The order fulfillment evaluation component 106 can further provide the output data 120' to at least one of, a device associated with the patient (e.g., patient device 216, such a personal computing device or network accessible patient management application accessed via the patient device 216) or a scheduling system (e.g., scheduling system 218, a RIS/EMR 220) configured to schedule the medical imaging exam for the patient in accordance with the recommendation information. The output data 120' can also be stored in the central database 116 and employed by the order fulfillment evaluation component 106 for tuning and updating one or more ML models employed by the order fulfillment evaluation component 106 to generate the output data 120'.

FIG. 3 illustrates a high-level block diagram of an example order fulfillment evaluation component 106, in accordance with one or more embodiments described herein. With reference to FIG. 3 in view of FIGs. 1 and 2, the order fulfillment evaluation component 106 can include several computer-executable components (e.g., that can be stored in memory 110 and executed by processing unit 112) to facilitate the operations described above. These computer-executable components can include but are not limited to, reception component 302, filtering component 304, quality component 306, AI component 308 (including training component 310, execution component 312, one or more ML models 314 and update analysis component 315), assessment component 316 (including selection component and ranking component 320), recommendation component 322 and scheduling component 324.

Generally, the reception component 302 can receive, collect or otherwise access and retrieve the input data 102 from the central database 116 and/or the respective input/output data sources 118_{1-N} providing the input data 102. For example, the reception component 302 can receive respective orders requesting performance of medical imaging exams on patients, the respective orders comprising order information 202 for the orders identifying a modality of the medical imaging exam, a clinical indication for the medical imaging exam, and an anatomical region to be captured in the medical imaging exam. The reception component 302 can also receive, access or otherwise collect additional relevant input data associated with the orders needed to generate the corresponding output data 120' for the respective orders (e.g., imaging device information, patient information 206, reference quality information 208, reference dose information 210 and historical medical imaging exam information 212).

In one or more embodiments, for an individual order to be fulfilled, the filtering component 304 can initially filter the available imaging devices included in the imaging devices information 204 based on the modality indicated for the exam in the order information. In this regard, the filtering component 304 can identify a subset of the available imaging devices that correspond to the modality (i.e., all imaging devices of the filtered subset are the same modality which is the modality prescribed for the exam in the order information). In some implementations, the filtering component 304 can also filter the available imaging devices based on other filtering criteria. For example, the other filtering criteria may be based on location of the patient (e.g., identified in the patient information 206) and identifying imaging devices withing a defined distance relative to the location, insurance coverage accepted (e.g., based on the patient's insurance coverage, as indicated in the patient information 206), a required device capability (e.g., as indicated based on the order information, such as high-resolution capability, contrast capability, a particular clinical application such as cardiac CT, or the like), and/or other filtering criteria indicated based on the order information 202 and/or the patient information 206 (e.g., urgency level of the exam and imaging device scheduling availability, patient imaging device preferences, and so on).

The AI component 308 employs one or more machine learning (ML) processes to predict or facilitate determining the output data 120' for the individual order. In some embodiments, the AI component 308 can employ one or more first machine learning processes to estimate measures of quality of the medical imaging exam capable of being generated by the different medical imaging devices of the same modality (i.e., those of the filtered subset) based on the order information (e.g., the ROI information, the clinical indication information and potentially other information included in the order) and relevant patient information associated with the patient for which the order is based (e.g., such as patient demographics, imaging constraints and preferences) and historical imaging exam information 212 regarding historical medical imaging exams performed by the different medical imaging devices that satisfy the order information (e.g., in terms of modality, ROI, clinical indication and any other criteria included in the order information) for similar patients in terms of demographics (e.g., patient size, gender, age, etc.), comorbidities imaging constraints and preferences. For example, using one or more ML processes, the AI component 308 can analyze resulting image quality metrics for same or similar exams performed for similar patients in terms of demographics represented in the historical medical imaging exam information 212. Based on the image quality metrics realized for the same or similar historical exams and patients, the AI component 308 can estimate expected measures of quality that can be achieved by the respective imaging devices of the filtered subset for the imaging exam prescribed for the new order.

The quality metrics for the respective historical medical imaging exams evaluated can include (but are not limited to): one or more SNR values, one or more CNR values, one or more resolution values (e.g., spatial resolution, contrast resolution, etc.), one or more measures regarding a quantitative or qualitive measure of artifacts, blurring and the like. The quality metrics for the respective historical medical imaging exams can also include one or more subjective quality measures, such as an interpretive measure of quality such as a quality score provided by the reviewing physician/radiologist such indicating whether and to what degree the quality of the images was diagnostically acceptable or not. Generally, medical imaging exams involve acquisition of a plurality of images, such as a series of images, different X-ray images from different perspectives of the patient, and the like. In this regard, the quality metrics for resulting images of respective historical exams can include or correspond to average quality metrics for the collective images of the respective exams. In some embodiments, these quality metrics can be predetermined and associated with the respective historical medical imaging exams as included in the historical medical imaging exam information 212. Additionally, or alternatively, the quality component 306 can determine the quality metrics for the historical exams based on analyzing the corresponding medical image data of the respective historical exams. Processing of the historical medical images by the quality component 308 to determine the quality metrics involves various steps, algorithms, and techniques, tailored to the specific imaging modality (e.g., MRI, CT, X-ray).

For example, in some embodiments, to evaluate spatial resolution, the quality component 306 can employ methods like the modulation transfer function (MTF). Spatial resolution refers to the ability of an imaging device to differentiate between two closely spaced objects. Higher spatial resolution allows for sharper and more detailed image. The MTF analyzes how well the imaging system can reproduce different spatial frequencies (details) from the object to the image. Another method that can be used by the quality component 306 to determine a measure of spatial resolution involves measuring the edge sharpness of structures in the image using an edge spread function (ESF). The ESF is used to calculate how quickly the intensity transitions from one region to another (e.g., from soft tissue to bone).

The quality component 306 can determine the CNR of respective historical medical images by measuring the difference in intensity between two regions (e.g., tumor vs. surrounding tissue) and dividing by the noise level. A higher CNR indicates better contrast resolution. Contrast resolution refers to the ability to distinguish between different tissues or structures based on differences in intensity or density. High contrast resolution or high CNR is essential for identifying subtle differences between soft tissues. The quality component 306 can also measure CNR as function of the histogram intensity of the image. A wider spread in intensity values across different structures suggests higher contrast resolution. For example, in MRI, the quality component 306 can analyze T1- or T2-weighted images to assess contrast resolution by looking at the differences between tissues like muscle and fat.

The quality component 306 can determine the SNR values for medical images by taking the ratio of the average signal intensity in a ROI to the standard deviation of the background noise. The SNR is a key factor in image quality. A higher SNR results in clearer images, while a lower SNR leads to grainy or noisy images. The quality component 306 can also determine the SNR in the frequency domain using the Fourier transform to separate the signal and noise components.

In some embodiments, the quality component 306 can measure an amount and/or severity of artifacts in the historical medical images using one or more automated artifact detection algorithms that scan the image for known types of artifacts (e.g., motion, streaks, or beam-hardening). These algorithms can identify and quantify artifacts based on their characteristic patterns. For example, artifacts often introduce spurious frequencies into the image data. These can be detected by the quality component 306 using frequency domain analysis (e.g., Fourier or wavelet transforms) to separate genuine image features from artifacts. For example, in MRI, phase-encoding artifacts caused by patient movement are detected by identifying repeating patterns across slices.

The quality component 306 can also process the historical medical images to determine quality metrics in terms of sharpness and edge detection. For example, the quality component 306 can employ algorithms like Sobel or Canny edge detectors to evaluate the sharpness of edges in an image. Sharp edges indicate better spatial resolution, whereas blurred edges suggest image degradation. Edge sharpness can be quantified by measuring the gradient of intensity across an edge. A steeper gradient indicates a sharper edge.

The quality component 306 can also perform histogram analysis to determine quality metrics representative of intensity range. A well-contrasted image will have a broad intensity range, whereas a poor image may show a compressed or skewed histogram. In some cases, the quality component 306 can binarize the images (converted to black and white) to evaluate the intensity thresholding performance, which is related to contrast and brightness.

The quality component 306 can also employ one or more pre-trained quality assessment models (e.g., neural network models and other types of machine learning models) to process the historical medical images and automatically generate quality metrics for them. These models are trained on large datasets of medical images with known quality metrics. For example, the one or more (pre-trained) quality assessment models can be configured to recognize patterns in images that correlate with high or low-quality metrics (e.g., identifying and quantifying blurred regions, detecting and measuring CNR contrast, detecting and measuring, SNR, artifacts and so on. In some embodiments, the one or more quality assessment models can be configured to generate an overall quality score for the collective images generated for a historical exam that represents a plurality of different quality metrics (e.g., CNR, SNR, spatial resolution, temporal resolution, artifacts, etc.).

In some embodiments, the estimated quality metrics determined by the AI component 308 for a new exam can represent an estimated minimum quality level (e.g., in terms of an overall quality score and/or individual metrics such as SNR, CNR, resolution, an amount/severity of artifacts) that can be achieved for the new exam by the respective imaging devices of the filtered subset, an estimated average quality level that can be achieved, and/or an estimated maximum quality level that can be achieved. In this regard, the quality of images generated via an imaging exam by a particular imaging device can depend on various factors, including the imaging device capabilities and the acquisition protocols/parameters used, and the technician performing the exam, which can vary. The acquisition protocols/parameters used also influence the amount of radiation dose exposed to the patient during the exam. Generally, the higher the radiation dose used as controlled as a function of the acquisition protocol/parameters used, the higher the resulting image quality.

In this regard, in medical imaging, particularly in modalities that use ionizing radiation, such as X-ray, CT, and nuclear medicine scans, there is a direct relationship between image quality and the radiation dose administered to the patient. The relationship is often a trade-off: improving image quality typically requires a higher radiation dose, while reducing the dose can degrade image quality. This balance is critical because radiation exposure carries risks, including the potential to increase the likelihood of cancer over time.

For example, SNR typically improves as the radiation dose increases. This is because a higher dose produces more X-ray photons, which leads to a stronger signal and less noise. Conversely, reducing the dose lowers the number of photons, increasing the noise and reducing the image quality. For example, in a CT scan, increasing the radiation dose produces sharper images with less noise, making it easier to detect fine anatomical details or subtle pathologies, such as small tumors or fractures. Similarly, contrast resolution or CNR is improved with a higher radiation dose because the increased number of photons provides a better distinction between tissues of varying densities. For example, in mammography, higher radiation doses may enhance contrast between breast tissue and potential abnormalities, such as tumors. However, optimizing the dose is crucial to minimize exposure while maintaining sufficient contrast to detect early signs of cancer. Spatial resolution is less directly impacted by radiation dose than SNR and contrast resolution. However, in practice, a higher dose can enhance the overall image quality, making it easier to resolve fine structures.

In this regard, for modalities such as X-ray, CT and nuclear medicine (e.g., PET and SPECT) the acquisition protocols and parameters are generally optimized with the goal of finding the right balance between minimizing radiation exposure and maintaining image quality sufficient for accurate diagnosis. Radiologists and medical physicists work together to adjust imaging protocols based on the clinical indicating, the ROI being imaged, the patient's health status and demographics (e.g., considering age, size, etc.) and the need for precise images. The primary consideration is to ensure that the image quality is diagnostically acceptable at the lowest possible radiation dose.

Thus, in various embodiments as applied to modalities that uses ionizing radiation (e.g., X-ray, CT and nuclear medicine), image quality can be defined good to bad based on an aggregate of observed noise metrics (e.g., SNR values or similar noise measure) for respective images included in the exam and the intensities of those noise as they relate to the recorded detail present on the imaging study. If the aggregate of the observed noise metrics supersedes the perceptibility as defined by the clinical indication for the exam, essentially meaning the presence of a lot of noise makes the image study uninterpretable, this would constitute a judgement by the reviewing party as bad image quality. The inverse would be applicable where noise is concerned, essentially less noise means better image quality. However, the quality of the study based on the judgement of image quality which is based on interpretability of the image study does not preclude the absence of noise. Rather, the presence of noise should be considered acceptable as meeting as low as reasonably achievable dosages. Optimal study quality should be defined as the lowest possible dose wherein an acceptable degree to which a person can assign qualitative meaning is achieved.

In some embodiments the one or more ML processes employed by the AI component 308 to estimate the quality metrics of resulting images capable of being generated by the respective imaging devices of the filtered subset based on the quality metrics for historical exams of the same type (e.g., in terms of modality, ROI, and clinical indication) for similar patients (e.g., in terms of relevant patient factors such as age, body size, gender, pregnancy status and health condition) demographics and health status) can include an assessment of the particular imaging protocols/parameters used, the technician performing the exam and the resulting radiation dose (for modalities such as X-ray, CT and nuclear medicine) exposed to the historical patients for the respective historical exams. In this regard, as opposed to assuming all imaging devices of the same modality represented in the imaging devices information 204 are theoretically capable of performing the imaging exam for the patient and achieving the acceptable diagnostic image quality level with an acceptable level or radiation (as applied to X-ray, CT, and nuclear medicine imaging exams) for the patient profile based on the theoretical acquisition protocol/parameter capabilities of the respective devices, the AI component 308 employs machine learning techniques to predict the best imaging device or devices for a new order based on historical performance of the respective imaging devices (e.g., based on the historical medical imaging data 212 and actual results of past exams of the same or similar type and for similar patients).

For example, in one or more embodiments, in association with assessing the expected performance of respective imaging devices of the same modality (e.g., filtered by the filtering component 304) for the new exam, for each imaging device the AI component 308 can estimate one or more maximum quality level metrics (or highest or best value) for the resulting images capable of being generated by the imaging device (e.g., based on historical quality metrics for past exams of the same or similar type performed by the imaging device for similar patients), along with the estimated radiation dose that can exposed to the patient (for modalities such as X-ray, CT and nuclear medicine) to achieve the corresponding maximum level quality metrics, and the corresponding acquisition protocols/parameters that can be used by the imaging device to achieve the maximum quality metrics (e.g., based on historical radiation dose reports for the past exams and corresponding acquisition protocols/parameters used). The AI assessment component 308 can also predict (e.g., based on historical performance of respective technicians that performing the past exams), the preferred technician or technicians that can perform the new exam to achieve the maximum quality. Additionally, or alternatively, the AI component 308 can estimate one or more average quality level metrics (or highest or best value) for the resulting images capable of being generated by the respective imaging devices of the subset along with the estimated radiation dose that can exposed to the patient to achieve the corresponding average level quality metrics and one or more corresponding acquisition protocols/parameters and technicians that can be used to achieve the average quality metrics.

In some implementations of these embodiments, the assessment component 316 can evaluate these results for the respective imaging devices of the same modality to determine the best imaging device or devices of the subset (and corresponding acquisition protocols/parameters and technicians) to be used for the new exam given one or more defined quality criteria and/or dose criteria for the new exam. Generally, in association with determining the best device or devices (and optionally the corresponding acquisition protocols/parameters and technicians) for the new exam, the goal of the assessment component 316 is to find the imaging device that can provide the best quality images or an acceptable quality level (for diagnostic purposes given the type of the new exam in terms of ROI, clinical indication and patient considerations) while providing the lowest radiation dose or an acceptable radiation dose level for the type of the exam and the patient (e.g., based on the patient's health status, size, age, gender, etc.).

For example, in some implementations, the selection component 318 can be configured to select the best device (or devices) of the subset which can provide the highest expected quality level without consideration of the corresponding expected radiation dosages. This strategy can be applied for imaging modalities that do not use radiation (e.g., MRI) as well as imaging modalities that do use radiation (e.g., X-ray, CT, and nuclear medicine). The recommendation component 322 can further generate recommendation data (e.g., output data 120') identifying the best device (as well as the technician(s) to be used and the corresponding acquisition protocols/parameters to be used). In some implementations, the ranking component 320 can rank the respective imaging devices in the subset based on expected quality in order from best device (highest expected quality) to least best device (lowest expected quality). The recommendation component 322 can further generate recommendation data (e.g., output data 120') identifying the devices of the subset and their ranking (as well as the technicians to be used and the corresponding acquisition protocols/parameters to be used to achieve the estimated maximum quality levels). Thus, when scheduling is performed (e.g., via scheduling component 324 and/or scheduling system 218), the scheduling component 234 and/or scheduling system 218 can be configured to schedule the new exam in accordance with the ranking by optimizing scheduling to select the higher ranked devices over lower ranked devices in consideration of other scheduling constraints and preferences, such as based on urgency level for the exam, patient location, and so on. In another example, the selection component 318 can be configured to select and the recommendation component 322 and be configured to recommend the top *N* (e.g., wherein *N* can be any predefined integer) ranked devices.

In other implementations, the selection component 318 can examine the maximum estimated quality metrics for the respective devices of the subset and select one or more of the best devices that can provide the highest quality level at the lowest radiation dose (e.g., using an optimization function that balances quality level and dose). For example, the selection component 318 can select a single best device from the subset that best balances achieving the highest quality level while minimizing radiation dose. The recommendation component 322 can further generate recommendation data (e.g., output data 120') identifying the best device (as well as the technician(s) to be used and the corresponding acquisition protocols/parameters to be used). In another example, the assessment component 316 can generate suitability scores for the respective devices of the subset using an optimization function that scores the respective devices based on weighing their maximum quality levels as a function of the corresponding dosages involved, wherein higher doses decrease the suitability score. The ranking component 320 can further rank the respective devices of the subset in order from best device to least best device as function of their suitability scores. The recommendation component 322 can further generate recommendation data (e.g., output data 120') identifying the devices of the subset and their ranking (as well as the technicians to be used and the corresponding acquisition protocols/parameters to be used to achieve the estimated maximum quality levels). Thus, when scheduling is performed (e.g., via scheduling component 324 and/or scheduling system 218), the scheduling component 234 and/or scheduling system 218 can be configured to schedule the new exam in accordance with the ranking by optimizing scheduling to select the higher ranked devices over lower ranked devices in consideration of other scheduling constraints and preferences, such as based on urgency level for the exam, patient location, and so on. In another example, the selection component 318 can be configured to select and the recommendation component 322 and be configured to recommend the top *N* (e.g., wherein *N* can be any predefined integer) ranked devices.

In other implementations, the one or more defined quality criteria can include or correspond to an acceptable quality level (e.g., in terms of an overall quality score and/or for individual quality metrics, such as SNR, CNR, spatial resolution, temporal resolution, etc.) for the medical images of the new exam. The acceptable quality level can be predefined for the particular exam type (e.g., in terms of modality, clinical indication and ROI) and optionally the patient profile (e.g., accounting for patient factors such as health status, age, gender, size, etc.) and included in the reference quality information 208 (and/or predicted by the AI component 308 using another ML process, as discussed below). With these implementations, the selection component can 318 can be configured select the best imaging device or devices in the subset for the new exam that can achieve the acceptable quality level at the lowest radiation dose(s). The recommendation component 322 can further generate recommendation data (e.g., output data 120') for the new exam identifying the best device and optionally the technician to be used and the corresponding acquisition protocols/parameters to be used. Additionally, or alternatively, for those imaging devices of the filtered subset whose estimated average and/or maximum quality level satisfy the acceptable quality criterion (or criteria) for the exam type and patient considerations, the ranking component 320 can rank the imaging devices based on the corresponding estimated radiation doses involved. The recommendation component 322 can further generate recommendation data (e.g., output data 120') identifying the devices and their ranking (as well as the technicians to be used and the corresponding acquisition protocols/parameters to be used). Thus, when scheduling is performed (e.g., via scheduling component 324 and/or scheduling system 218), the scheduling component 234 and/or scheduling system 218 can be configured to schedule the new exam in accordance with the ranking by optimizing scheduling to select the higher ranked devices over lower ranked devices in consideration of other scheduling constraints and preferences, such as based on urgency level for the exam, patient location, and so on. In another example, the selection component 318 can be configured to select and the recommendation component 322 and be configured to recommend the top *N* ranked devices that can achieve the acceptable quality criterion.

Additionally, or alternatively, as applied to modalities like X-ray, CT and nuclear medicine, the assessment component 316 can be configured to select the best device or devices of the subset based on a dose criterion, such as an acceptable dose level or dose level range for the type of imaging exam of the new order and optionally the patient (e.g., based on the patient size, age, gender, health status, comorbidities, etc.). The acceptable quality level can be predefined for the particular exam type (e.g., in terms of modality, clinical indication and ROI), and optionally the profile of the patient and included in the reference dose information 210). With these implementations, the selection component can 318 can be configured select the best imaging device or devices in the subset for the new exam and corresponding acquisition protocols/parameters and technicians to be used that can achieve the acceptable dose level or level range at the highest image quality level. The recommendation component 322 can further generate recommendation data (e.g., output data 120') for the new exam identifying the best device and optionally the technician to be used and the corresponding acquisition protocols/parameters to be used. Additionally, or alternatively, for those imaging devices and acquisition protocol/parameter combinations of the filtered subset whose expected dose levels satisfy the acceptable dose criterion for the exam type and patient considerations, the ranking component 320 can rank the imaging devices based on the corresponding estimated quality levels achievable. The recommendation component 322 can further generate recommendation data (e.g., output data 120') identifying the devices and their ranking (as well as the technicians to be used and the corresponding acquisition protocols/parameters to be used). Thus, when scheduling is performed (e.g., via scheduling component 324 and/or scheduling system 218), the scheduling component 234 and/or scheduling system 218 can be configured to schedule the new exam in accordance with the ranking by optimizing scheduling to select the higher ranked devices over lower ranked devices in consideration of other scheduling constraints and preferences, such as based on urgency level for the exam, patient location, and so on. In another example, the selection component 318 can be configured to select and the recommendation component 322 and be configured to recommend the top (N ranked devices that can achieve the acceptable dose criterion.

Still in other embodiments, based on reception of a new order, the AI component 308 can determine or infer (i.e., predict using one or more ML processes) one or more acceptable quality metrics for resulting images to be obtained via the exam based on the exam type (e.g., in terms of modality, ROI, and clinical indication for the exam) and the patient (e.g., accounting for patient factors such as health status, age, gender, size, etc.). For example, in some embodiments, the one or more acceptable quality metrics can be predefined and provided via the reference quality information 208. In other embodiments, the AI component 308 can employ one or more ML processes to predict the acceptable quality metrics for the exam type and the patient based on analysis of historical quality information (included in the historical medical imaging exam information 212) for same or similar exam types and patient profiles and feedback associated with the respective historical exams indicating whether and to what degree the historical quality metrics were diagnostically acceptable. For example, such feedback can be associated with the historical exams as provided by interpreting clinicians, such as in corresponding radiology reports, clinician notes or the like. Such feedback can also be gleaned based on medical records associated with the patients/exams indicating whether one or more repeat exams were needed and indicating whether the resulting diagnoses were accurate. To this end, the AI component 308 can learn and define the reference quality information 208 for different exam types and different patient profiles (e.g., wherein the different patient profiles account for different patient factors including demographic factors and health status factors).

With these embodiments, given the one or more acceptable quality metrics for a new imaging order, the AI component 308 can employ one or more machine learning processes to predict whether respective devices of the filtered subset can achieve the one or more acceptable quality metrics based on the historical quality metrics for same or similar exam types and patient profiles. For example, for each imaging device of the subset, the AI component 308 can predict whether the imaging device is capable of performing the exam and achieving the one or more acceptable quality measures. As the quality of the resulting images depend on the acquisition protocols/parameters used and the technician performing the exam, for each imaging device that can achieve the acceptable quality metrics, the AI component 308 can also predict (e.g., based on the historical information) one or more acquisition protocols/parameters and corresponding technicians that can be used to achieve the acceptable quality metrics. For imaging modalities such as X-ray, CT and nuclear medicine, the AI component 308 can also estimate the corresponding radiation doses involved. In other implementations, using one or more ML processes, the AI component 308 can predict the particular acquisition protocols/parameters and technician combinations that can be used by the imaging devices to achieve the acceptable quality metrics at the lowest radiation dose.

With these embodiments, the selection component 318 can be configured to select one or more of the devices of the subset as the best device or devices for the exam which can achieve the acceptable quality metrics, or that can achieve the acceptable quality metrics at the lowest dose or doses, and the corresponding acquisition protocols/parameters and technicians to be used. The recommendation component 322 can further generate recommendation data (e.g. output data 120') identifying the selected best device or devices of the subset (as optionally ranked by the ranking component 320 based on corresponding lowest dose, wherein the lower the corresponding dose the higher the ranking).

Additionally, or alternatively, in embodiments in which the selection component 318 selects the one or more best imaging devices based on their estimated quality measures for the exam satisfying a defined quality criterion, such as an acceptable measure of quality for the medical imaging exam, the selection component 318 can also select the one or more best imaging devices based on a dose criterion, an acquisition protocol criterion and/or or an acquisition parameter criterion associated with the medical imaging exam. For example, in some embodiments, the AI component 308 can employ one or more first machine learning processes or one or more second machine learning processes to estimate, based on the order information and the historical imaging exam information, radiation doses capable of being exposed to the patient via the different medical imaging devices in association with performing the medical imaging exam and achieving the acceptable measure of quality, wherein the selecting the subset is further based on respective radiation doses for the subset satisfying a radiation dose criterion (e.g., an acceptable radiation dose level for the type of imaging exam and the patient). In some implementations, the employing the one or more first machine learning processes or the one or more second machine learning processes further comprises determining respective acquisition protocols capable of being used by the respective medical images devices in association with performing the medical imaging exam and achieving the acceptable measure of quality with the respective radiation doses that satisfy the radiation dose criterion, and wherein the generating the recommendation information further comprises including acquisition protocol information with the recommendation information identifying the respective acquisition protocols for the respective devices. In some implementations of these embodiments, the selection component 318 can select the subset (i.e., the one or more optimal imaging devices) based on the respective acquisition protocols for the subset satisfying an acquisition protocol criterion. For example, the acquisition protocol criterion can include or correspond to a defined acquisition protocol criterion included in the order information (e.g., contrast vs. non-contrast) and/or defined for the type of the imaging exam and/or the patient.

In another example, in association with employing the one or more first machine learning processes or the one or more second machine learning processes, the AI component 308 can determine respective acquisition parameters capable of being used by the respective imaging devices in association with performing the medical imaging exam and achieving the acceptable measure of quality with the respective radiation doses for the subset that satisfy the radiation dose criterion, and wherein the generating the recommendation information further comprises including acquisition parameter information with the recommendation information identifying the respective acquisition parameters for the subset. In some implementations of these embodiments, the selection component 318 can select the subset of best imaging devices based on the respective acquisition parameters for the subset satisfying an acquisition parameter criterion. For example, the acquisition protocol criterion can include or correspond to a defined acquisition protocol criterion included in the order information and/or defined for the type of the imaging exam and/or the patient.

In this regard, the AI component 308 can employ principles of artificial intelligence and ML to facilitate predicting the best imaging device or devices of the same modality for fulfilling a new order based on learning patterns, correlations and relationships between how different factors (e.g., exam type, patient factors, acquisition protocols/parameters, technicians, etc.) influence resulting image quality and radiation dose as learned from the input data 102 (e.g., the historical medical imaging exam information 212, the imaging devices information 204, the patient information 206, the reference quality information 208 and the reference dose information 210). The AI component can also employ AI and ML to learn and defined the reference quality information 208 for different imaging exam types and patient profiles.

The AI component 308 can perform such learning explicitly or implicitly. Learning and/or determining inferences by the AI component 308 can facilitate identification and/or classification of different patterns associated with the input data 102, determining one or more rules associated with the input data 102 and/or determining one or more relationships between how different factors in the input data 102 (e.g., exam type, patient factors, acquisition protocols/parameters, technicians, etc.) influence resulting image quality and radiation dose and the acceptable quality measures for different exam types an patient profiles. The AI component 308 can also employ an automatic classification system and/or an automatic classification process to facilitate such learning. For example, the AI component 308 can employ a probabilistic and/or statistical-based analysis (e.g., factoring into the analysis utilities and costs) to learn patterns, correlations and relationships between how different factors (e.g., exam type, patient factors, acquisition protocols/parameters, technicians, etc.) influence resulting image quality and radiation dose. The AI component 308 can also employ a probabilistic and/or statistical-based analysis to learn the reference quality information based on the historical medical imaging exam information 212 and associated feedback regarding acceptable quality measures for past exams. The AI component 308 can employ, for example, a support vector machine (SVM) classifier to perform such learning

Additionally, or alternatively, the AI component 308 can employ other classification techniques associated with Bayesian networks, decision trees and/or probabilistic classification models. Classifiers employed by the AI component 308 can be explicitly trained (e.g., via a generic training data) as well as implicitly trained (e.g., via observing user behavior, receiving extrinsic information). For example, with respect to SVM's that are well understood, SVM's are configured via a learning or training phase within a classifier constructor and feature selection module. A classifier is a function that maps an input attribute vector, x = (x1, x2, x3, x4, xn), to a confidence that the input belongs to a class - that is, f(x) = confidence(class).

In this regard, the AI component 308 can employ any suitable machine-learning based techniques, statistical-based techniques and/or probabilistic-based techniques. The AI component 308 can additionally or alternatively employ a reduced set of factors (e.g., an optimized set of factors) to facilitate providing a most accurate machine learning model for predicting census in respective medical inpatient units. For example, the AI component 308 can employ expert systems, fuzzy logic, SVMs, Hidden Markov Models (HMMs), greedy search algorithms, rule-based systems, Bayesian models (e.g., Bayesian networks), neural networks, other nonlinear training techniques, data fusion, utility-based analytical systems, systems employing Bayesian models, etc. In another aspect, the AI component 308 can perform a set of machine learning computations associated with the input data 102. For example, the AI component 308 can perform a set of clustering machine learning computations, a set of decision tree machine learning computations, a set of instance-based machine learning computations, a set of regression machine learning computations, a set of regularization machine learning computations, a set of rule learning machine learning computations, a set of Bayesian machine learning computations, a set of deep Boltzmann machine computations, a set of deep belief network computations, a set of convolution neural network computations, a set of stacked auto-encoder computations and/or a set of different machine learning computations.

The one or more patterns, correlations and relationships between how different factors (e.g., exam type, patient factors, acquisition protocols/parameters, technicians, etc.) influence resulting image quality and radiation dose as learned from the input data 102 (e.g., the historical medical imaging exam information 212, the imaging devices information 204, the patient information 206, the reference quality information 208 and the reference dose information 210), as well as the reference quality information 208 as learned and defined by the AI component 308 can be stored in a machine learning database (e.g., central database 116) and/or the memory 110.

In this regard, to facilitate predicting the best imaging device or devices of the same modality for fulfilling a new order, the AI component 308 can employ one or more ML processes. In some embodiments, the AI component 308 can employ one or more first machine learning processes to estimate measures of quality of the medical imaging exam capable of being generated by the different medical imaging devices of the same modality based on the order information and historical imaging exam information regarding historical medical imaging studies performed by the different medical imaging devices that satisfy the order information. The estimated measures of quality can include average quality measures and/or highest quality measures. The one or more first machine learning processes can involve learning patterns, correlations and relationships between how different factors (e.g., exam type, patient factors, acquisition protocols/parameters, technicians, etc.) influence resulting image quality as learned from the historical medical imaging exam information 212, the imaging devices information 204, the patient information 206, the reference quality information 208 and the reference dose information 210. Based on the learned patterns, the AI component 308 can estimate the measures of quality of the new medical imaging exam capable of being generated by the different medical imaging devices of the same modality.

In some embodiments, the one or more first machine learning processes can involve training (e.g., via training component 310) one or more ML models 314 to estimate the quality measures (and optionally the corresponding acquisition protocols/parameters and technicians to be used) based on the historical medical imaging exam information 212. With these embodiments, the one or more ML models 314 can include or correspond to quality one or more image quality models. The execution component 312 can apply one or more applicable trained versions of the image quality models to input data 102' for a new order to estimate the measures of quality of the new medical imaging exam capable of being generated by the different medical imaging devices of the same modality (and optionally the corresponding acquisition protocols/parameters and technicians to be used).

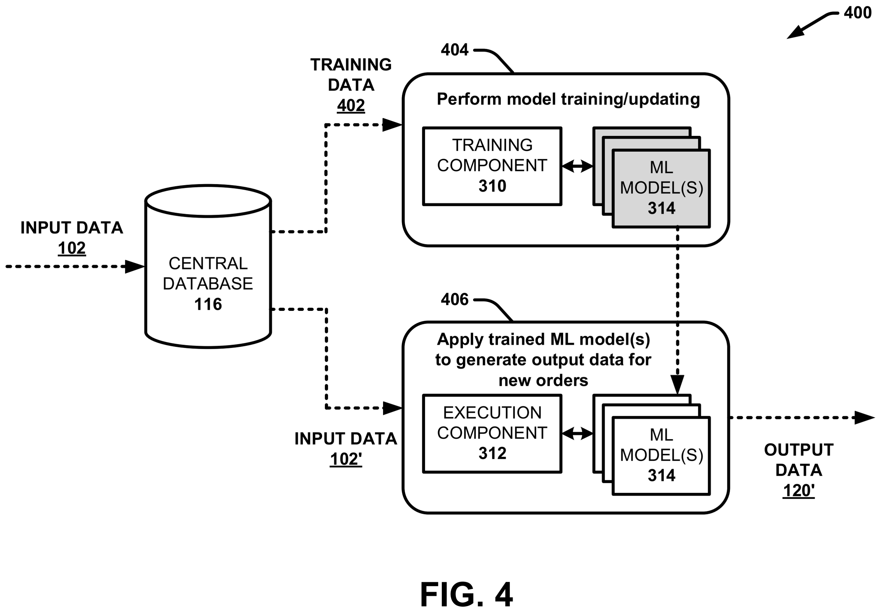

In this regard, FIG. 4 illustrates an example machine learning process 400 that facilitates predicting the best medical imaging device(s) for fulfilling an imaging exam order, in accordance with one or more embodiments described herein. With reference to FIG. 4 in view of FIGs. 1-3, in accordance with process 400, the input data 102 can be received and stored in a central database 116 as it is generated by the corresponding input/output data sources. This input data 102 can include new orders as they are prescribed and entered into a RIS (or the like) as well as imaging devices information 204, patient information 206, reference quality information 208, reference dose information 210 and historical medical imaging exam information 212. In accordance with process 400, the AI component 308 can employ some or all of the input data 102 excluding new orders to be fulfilled as training data 402 for ML model 314 development (e.g., initial training) and updating over time. In this regard, at 404, the training component 310 employs the training data 402 to train (and sometimes update) one or more ML models 314. The ML models 314 are illustrated with gray in process 400 to indicate they are undergoing training and/or updating. Corresponding trained and/or updated versions of the ML models 314 are indicated with white fill. In this regard, one trained/updated, at 406, the execution component 312 applies input data 102' for a new order as input to the one or more ML models 314 to generate the output data 120' for the new order.

To this end, as applied to the aforementioned image quality models, the input data 102' for the new order can include the order information which describes the modality, the ROI and the clinical indication. The input data 102' can also include relevant patient parameters of the patient for which the order is made

(e.g., as included in the order information or corresponding patient information 206). For example, the relevant patient parameters can include but are not limited to, the patient's health status, size, age, gender, comorbidities and other relevant patient parameters discussed herein. The input data 102' can also include the corresponding imaging device information (included in imaging devices information 204) for the filtered subset of imaging devices having the same modality as that prescribed in the order information. The output data 120' can include, for each imaging device, expected measures of quality capable of being generated by the imaging device for the new exam. The expected measures of quality can represent one or more average quality measures and/or one or more highest quality measures (e.g., an overall quality score, and/or individual quality metric values, such as an SNR value, a CNR value, a temporal resolution value, a spatial resolution value, an artifact level value, and so on).

In accordance with these embodiments, the training component 310 can train the one or more quality models to generate such output data 120' based on feeding the one or more quality models input training data samples corresponding to different variations of the input data' as extracted from or created from the historical medical imaging exam information 212 and using historical quality measures known for the past exams as the ground truth (GT) information. The different variations can reflect a plurality of different exams of the same or similar type (e.g., with respect to modality, ROI, and clinical indication) for similar patients. The different variations can also reflect a plurality of different exams exam types for different patient profiles. The training process used can include or correspond to a supervised machine learning process. The type of the ML models 314 can vary and include or correspond to deep learning (DL) models, neural network models, deep neural network models (DNNs), convolutional neural network models (CNNs), generative adversarial neural network models (GANs), transformer models, and the like.

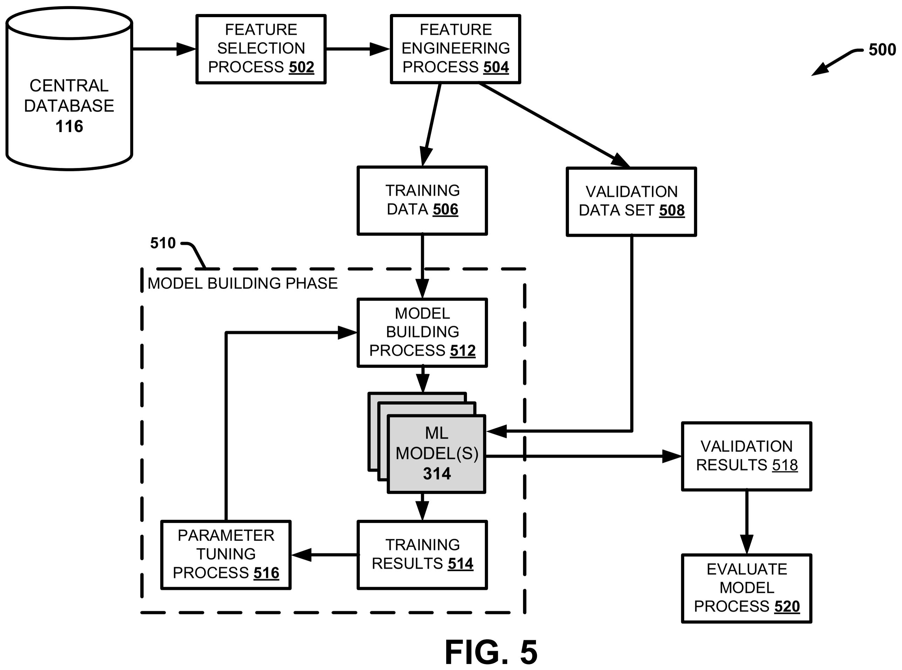

FIG. 5 illustrates an example training process 500 for training one or more machine learning (ML) models to predict or facilitate predicting the best medical imaging device(s) for fulfilling an imaging exam order, in accordance with one or more embodiments described herein. In various embodiments, the training component 310 can employ training process 500 in association with training and/or updating the one or more ML models 314 at 404 of process 400.

In this regard, training process 500 can includes a model building phase 510 for a machine learning process. The model building phase 510 can employ training data 506 and/or validation data set 508 as extracted from the information (e.g., input data 102) included in the central database 116. In an embodiment, training data 506 and/or validation data set 508 can be generated via a features selection process 502 and/or a feature engineering process 504. The feature engineering process 504 can employ a machine learning process to generate the training data 506 and/or validation data set 508. For example, the feature engineering process 504 can employ a training phase of a machine learning process to generate the training data 504. Additionally, or alternatively, the feature engineering process 504 can employ a prediction phase of a machine learning process to generate the validation data set 508. In certain embodiments, all or some of the information included in the central database 116 can be employed by a feature selection process 502. The feature selection process 502 can select one or more features to build one or more ML models 314 via the feature engineering process 504. In another embodiment, the model building phase 510 can include a model building process 512 and the model building process 512 can build the one or more ML models 314. For example, the model building process 512 can build an ML model based on the training data 506 and/or the validation data set 508. The one or more ML models 314 can include the any of the various image quality models, acquisition protocol/parameter models, and/or requisite image quality models discussed herein.

In an aspect, during the model building process 512, the one or more ML models 314 can provide training results 514. Furthermore, a parameter tuning process 516 can be performed based on the training results 514 to tune one or more parameters for the model building process 512. In another aspect, the one or more ML models 314 can provide validation results 518 to validate the one or more ML models 314. The validation results 518 can be employed by an evaluate model process 520 to evaluate one or more metrics for the one or more ML models 314.

With reference to FIGs. 1-5 additionally, or alternatively, as applied to modalities such as X-ray, CT and nuclear medicine, to facilitate predicting the best imaging device or devices of the same modality for fulfilling a new order, the AI component 308 can employ one or more second machine learning (and/or the one or more first machine learning process) to estimate, based on the order information and the historical imaging exam information, radiation doses capable of being exposed to the patient via the different medical imaging devices of the same modality in association with performing the medical image exam and achieving one or more acceptable measure of quality defined or inferred for the new imaging exam (e.g., as included in the reference quality information 208). In this regard, the one or more first and/or second machine learning processes can involve learning patterns, correlations and relationships between how different factors (e.g., exam type, patient factors, acquisition protocols/parameters, technicians, etc.) influence resulting dosage levels of the historical exams which satisfy the acceptable quality measures, as learned from the historical medical imaging exam information 212, the imaging devices information 204, the patient information 206, and the reference dose information 210. Based on the learned patterns, the AI component 308 can estimate the radiation doses capable of being exposed to the patient via the different medical imaging devices of the same modality (and optionally the corresponding acquisition protocols/parameters and technicians to be used) in association with performing the medical image exam and achieving one or more acceptable measure of quality.

With these embodiments, the one or more first and/or second machine learning processes can involve training (e.g., via training component 310) the aforementioned image quality models and/or one or more second image quality models to estimate the expected, average or minimum radiations doses (and optionally the corresponding acquisition protocols/parameters and technicians to be used) under the quality constraints based on the historical medical imaging exam information 212. In other words, the ML models 314 can include one or more image quality models configured to estimate the dose information under the constraint imparted by the acceptable quality metrics tailored for the type of the exam and the profile of the patient. The execution component 312 can apply one or more applicable trained versions of theses models image quality models to input data 102' for a new order to estimate the radiation doses of the new medical imaging exam capable of being generated by the different medical imaging devices of the same modality, (and optionally the corresponding acquisition protocols/parameters and technicians to be used). Additional details regarding these image image quality models and the training process are described infra.

With these embodiments, the input data 102' for the one or more (second) image quality models for the new order can include the order information which describes the modality, the ROI and the clinical indication. The input data 102' can also include relevant patient parameters of the patient for which the order is made (e.g., as included in the order information or corresponding patient information 206). The input data 102' can also include the corresponding imaging device information (included in imaging devices information 204) for the filtered subset of imaging devices having the same modality as that prescribed in the order information. The input data 102' can additionally include the applicable quality metric(s) for the new order. The output data 120' can include, for each imaging device, information indicating whether each imaging device is capable of achieving the applicable quality metrics. For those imaging devices that are cable (which may include some or all of the imaging devices evaluated), the output data 120' can also include the radiation doses expected (e.g., the average radiation doses and/or the minimum radiation doses) and optionally the corresponding acquisition protocols/parameters and technicians to be used).

In accordance with these embodiments, the training component 310 can train the one or more (second) image quality models to generate such output data 120' based on feeding the one or more (second) image quality models input training data samples corresponding to different variations of the input data' as extracted from or created from the historical medical imaging exam information 212 and using historical dose measures known for the past exams as the ground truth (GT) information. The different variations can reflect a plurality of different exams of the same or similar type (e.g., with respect to modality, ROI, and clinical indication) for similar patients and under the same or similar image quality constraint. The different variations can also reflect a plurality of different exams exam types for different patient profiles. The training data samples can also include and account for the respective imaging protocols/parameters and technicians used for the historical exams. The training process used can include or correspond to training process 500.

Still in other embodiments, as applied to modalities such as X-ray, CT and nuclear medicine, to facilitate predicting the best imaging device or devices of the same modality for fulfilling a new order, the AI component 308 can employ one or more estimate, based on the order information and the historical imaging exam information, radiation doses capable of being exposed to the patient via the different medical imaging devices of the same modality in association with performing the medical image exam and achieving a radiation dose criterion defined for the new imaging exam (e.g., as included in the reference dose information 210). With these embodiments, the one or more machine learning processes can involve training (e.g., via training component 310) the aforementioned image quality models and/or one or third image quality models to estimate the average and/or highest quality measure(s) (and optionally the corresponding acquisition protocols/parameters and technicians to be used) to be achieved by the respective imaging devices of the same modality under the dose constraint.

With these embodiments, the input data 102' for the one or more (third) image quality models for the new order can include the order information which describes the modality, the ROI and the clinical indication. The input data 102' can also include relevant patient parameters of the patient for which the order is made (e.g., as included in the order information or corresponding patient information 206). The input data 102' can also include the corresponding imaging device information (included in imaging devices information 204) for the filtered subset of imaging devices having the same modality as that prescribed in the order information. The input data 102' can additionally include the dose constraint for the new order. The output data 120' can include, for each imaging device, the average and/or highest quality metrics expected for the resulting images that satisfy the dose constraint, and optionally the corresponding acquisition protocols/parameters and technicians to be used.

In accordance with these embodiments, the training component 310 can train the one or more (third) image quality models to generate such output data 120' based on feeding the one or more (image) image quality models input training data samples corresponding to different variations of the input data' as extracted from or created from the historical medical imaging exam information 212 and using historical quality measures known for the past exams as the ground truth (GT) information. The different variations can reflect a plurality of different exams of the same or similar type (e.g., with respect to modality, ROI, and clinical indication) for similar patients and that satisfy the dose constraint. The different variations can also reflect a plurality of different exams exam types for different patient profiles. The training data samples can also include and account for the respective imaging protocols/parameters and technicians used for the historical exams. The training process used can include or correspond to training process 500.

In other embodiments, the one or more ML models 314 can additionally, or alternatively include one or more acquisition protocol/parameter quality models trained to predict one or more possible acquisition protocols/parameters that can be used by the imaging devices of the same modality to achieve the quality constraint for the exam (e.g., as included in the reference quality information 208 or estimated via the AI component 308) and/or a dose constraint for the new exam (e.g., as defined in the reference dose information 210).

In some embodiments, the one or more ML models 314 can also include one or more requisite image quality models configured to estimate the requisite or applicable quality measure(s) for a new imaging exam. With these embodiments, the training component 310 can train one or more requisite image quality models to estimate required or preferred quality metrics to be achieved for the images of the new exam based on the exam type (e.g., modality, ROI and clinical indication) and the patient (e.g., considering relevant patient factors such as health status, age, gender, size, etc.). With these embodiments, the input data 102' for the one or more requisite image quality models for the new order can include the order information which describes the modality, the ROI and the clinical indication. The input data 102' can also include relevant patient parameters of the patient for which the order is made (e.g., as included in the order information or corresponding patient information 206). The output data includes one or more requisite or applicable quality measure(s) for a new imaging exam.

In accordance with these embodiments, the training component 310 can train the one or more requite image quality models to estimate the requisite or applicable quality measure based on feeding the requisite image quality models input training data samples corresponding to different variations of the input data as extracted from or created from the historical medical imaging exam information 212 and using historical quality measures known for the past exams (e.g., actual observed quality metric values) and feedback (e.g., provided by one or more entities that reviewed the images, and/or other feedback such as information indicating whether a repeat scan was needed) regarding whether and to what historical quality measures were diagnostically acceptable or not as the ground truth (GT) information. The different variations can reflect a plurality of different exams accounting for different exam types and patient profiles. The training process used can include or correspond to training process 500.

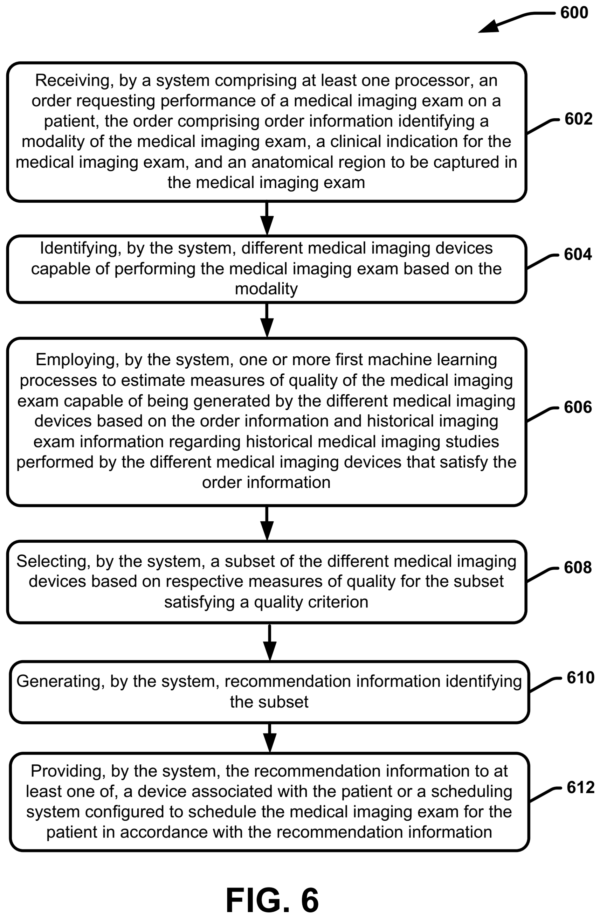

FIG. 6 illustrates an example computer-implemented method 600 for predicting and recommending the best medical imaging device(s) for fulfilling an imaging exam order, in accordance with one or more embodiments described herein. With reference to FIG. 6 in view of FIGs. 1-5, method 600 corresponds to an example method that can be performed by system 100 in accordance with an embodiment.

At 602, method 600 comprises receiving (e.g., via reception component 302), by a system comprising at least one processor (e.g., system 100), an order requesting performance of a medical imaging exam on a patient, the order comprising order information identifying a modality of the medical imaging exam, a clinical indication for the medical imaging exam, and an anatomical region to be captured in the medical imaging exam. At 604, method 600 comprises identifying, by the system, different medical imaging devices capable of performing the medical imaging exam based on the modality (e.g., via filtering component 304). At 606, method 600 comprises employing, by the system, one or more first machine learning processes to estimate measures of quality of the medical imaging exam capable of being generated by the different medical imaging devices based on the order information and historical imaging exam information regarding historical medical imaging exams performed by the different medical imaging devices that satisfy the order information (e.g., via AI component 308). At 608, method 600 comprises selecting, by the system, a subset (e.g., the subset comprising one or more) of the different medical imaging devices based on respective measures of quality for the subset satisfying a quality criterion (e.g., via selection component 318).

For example, in some implementations, the quality criterion may comprise one or more acceptable (or required/preferred) quality measures or quality measure values (e.g., an overall quality score and/or individual quality metric values, such as an acceptable SNR metric, an acceptable CNR metric, an acceptable spatial resolution, an acceptable temporal resolution metric, an acceptable artifact metric, and so on) for the exam tailored to the exam type and/or the patient. In another example, the quality criterion may include or correspond to a highest quality level, such that the selection component selects the top imaging device or the top N imaging devices with the highest quality measures.

At 610, method 600 comprises generating, by the system, recommendation information identifying the subset (e.g., via recommendation component 322). At 612, method 600 comprises providing, by the system (e.g., via recommendation component 322), the recommendation information to at least one of, a device associated with the patient (e.g., patient device 216) or a scheduling system (e.g., scheduling system 218 and/or RIS/EMR 220) configured to schedule the medical imaging exam for the patient in accordance with the recommendation information.

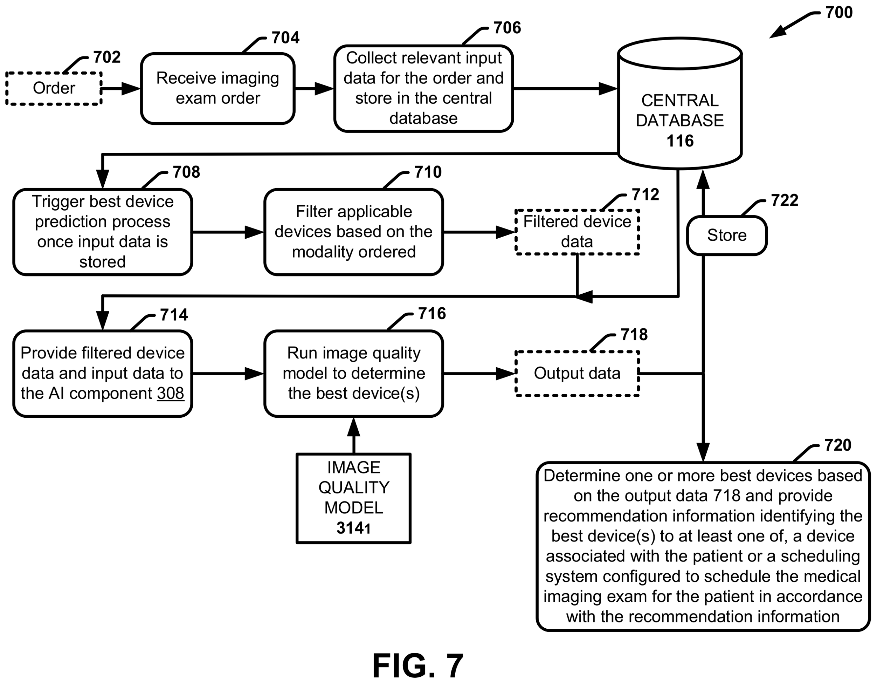

FIG. 7 illustrates a flow-diagram of another example computer-implemented method 700 for predicting and recommending the best medical imaging device(s) for fulfilling an imaging exam order, in accordance with one or more embodiments described herein. With reference to FIG. 7 in view of FIGs. 1-5, method 700 corresponds to an example method that can be performed by system 100 in accordance with an embodiment.

In accordance with method 700, at 704 the system (e.g., system 100) receives an imaging exam order 702 for a patient to be scheduled and performed. For example, the system can receive the order 702 in response to entering of the order into a RIS connected to the computing device 104 or in another manner. The order 702 may thus correspond to an electronic order entry into the RIS or a similar source (e.g., included in the input/output data sources 118_{1-N}). In some implementations, as orders are issued for patients and entered into the RIS, they can be populated into and stored in the central database 116. The order 702 can comprise information identifying the patient, the modality, a clinical indication for the medical imaging exam, and an anatomical region (i.e., ROI) to be captured in the medical imaging exam. In some implementations, the order may comprise relevant information about the patient, such as information indicating the patient's health status, size, gender, age, and the like. In other implementations, this information can be collected/extracted from the patient's medical records (e.g., in an EMR, EHR, or another source) at 706 based on the patient's ID as included in the order 702.

At 706, the system can collect (e.g., via reception component 302) relevant input data for the order and stores it in the central database 116. For example, the relevant input data can include the order information and the relevant patient information for the patient. The relevant input data also includes the imaging devices information 204, the reference quality information quality information 208, and the reference dose information 210. Additionally, or alternatively, this information may already be included in the central database 116 and/or accessed by the computing device 104 at their corresponding sources.

At 708, the system triggers the best device prediction process once the input data is stored in the central database 116. At 710, the best device prediction process can involve initially filtering the applicable devices (e.g., included in the imaging devices information 204) based on the modality ordered for the exam (e.g., X-ray, CT, nuclear medicine, MRI, and others) to generate filtered device data 712. The filtered device data 712 represents a collection of imaging devices of the same modality, that is the modality ordered for the exam. At 714, the filtered device data 712 and the input data (e.g., the order information and the relevant patient information) are provided to the AI component 308. At 716, the execution component 312 runs an image quality model 314₁ to determine the best device(s). For example, the image quality model 314₁ can correspond to one or more of the ML models 314.

In an embodiment, the image quality model 314₁ can be configured to process input data comprising the order information, the patient parameters and the filtered device data, and generates output data 718 comprising estimated quality measures for the respective filtered devices regarding estimated measures of quality for the exam images capable of being generated by the respective devices. The estimated quality measures can include, for each device, one or more average quality measures and/or one or more highest quality measures. In some implementations, the output data 718 generated by the image quality 314₁ can include corresponding acquisition parameters/protocols and technicians that can be used by the respective devices to obtain the estimated quality measures. In some implementations, (as applied to X-ray, CT, and nuclear medicine modalities), the output data 718 can also include estimated dose levels expected to be exposed to the patient via the respective imaging devices in association with obtaining the estimated quality measures (e.g., dose for the average quality measures and dose for the highest quality measures).

At 720, the assessment component 316 can determine one or more best devices for the exam based on the output data and provide recommendation information (e.g., output data 120') identifying the best device(s) to at least one of, a device associated with the patient or a scheduling system configured to schedule the medical imaging exam for the patient in accordance with the recommendation information. The recommendation information can include also include the output data 718 associated with the best devices (e.g., information regarding estimated quality measures, corresponding acquisition protocols/parameters, and corresponding estimated doses). In this regard, at 720 the assessment component 316 can select (e.g., via selection component 318) one or more of the imaging devices in the filtered device group based on the results of the image quality model 314₁. For example, the selection component 318 can select the one or more best devices based on the best devices satisfying a quality criterion and/or a dose criterion (where applicable depending on the modality).

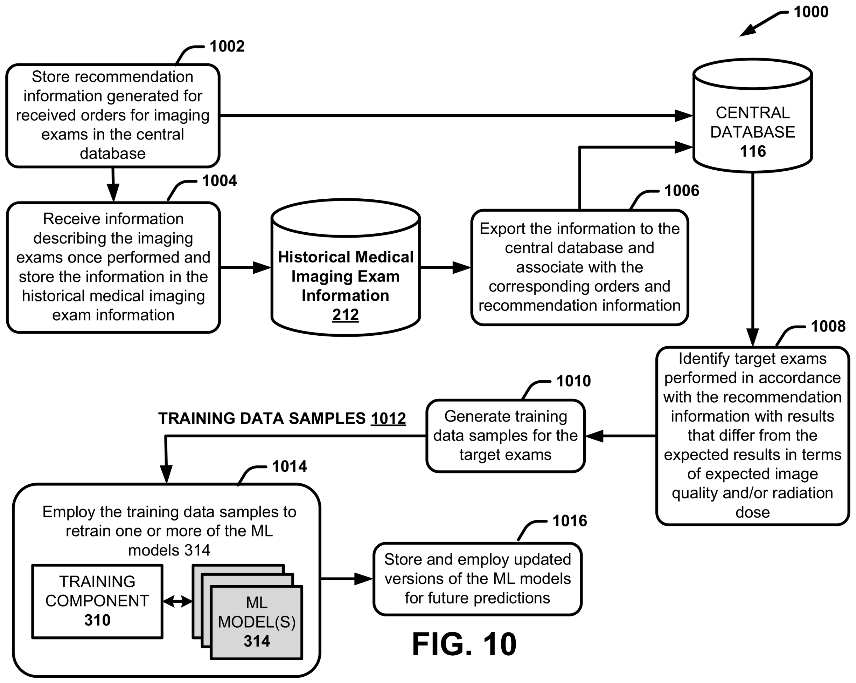

The recommendation information can also be stored in the central database 116 and used by the training component 310 to update (e.g., refine/tune) the image quality model 314₁ after the imaging exam has been performed for the patient, as illustrated in FIG. 10. In this regard, the recommendation information can be associated with the order for the patient in the central database 116. After the exam has been performed using the best device (or one of the best devices), information about the details of the exam and the actual results with respect to resulting image quality and whether the quality was diagnostically acceptable, acquisition parameters/protocols used, and radiation dose exposed to the patient can be determined (e.g., via the DICOM data, the image quality component 306, corresponding dose reports associated with exam, the radiology report, and so on) and represent a historical exam entry included in the historical imaging exam information 212. As additional new historical exam entries coupled with recommendation information generated by the order fulfillment component 106 are obtained and aggregated, the training component 310 can update the image quality model 314₁ based on differences between the recommendation information and the actual results and using the new historical exam entries as training samples, as described with reference to FIG. 10.

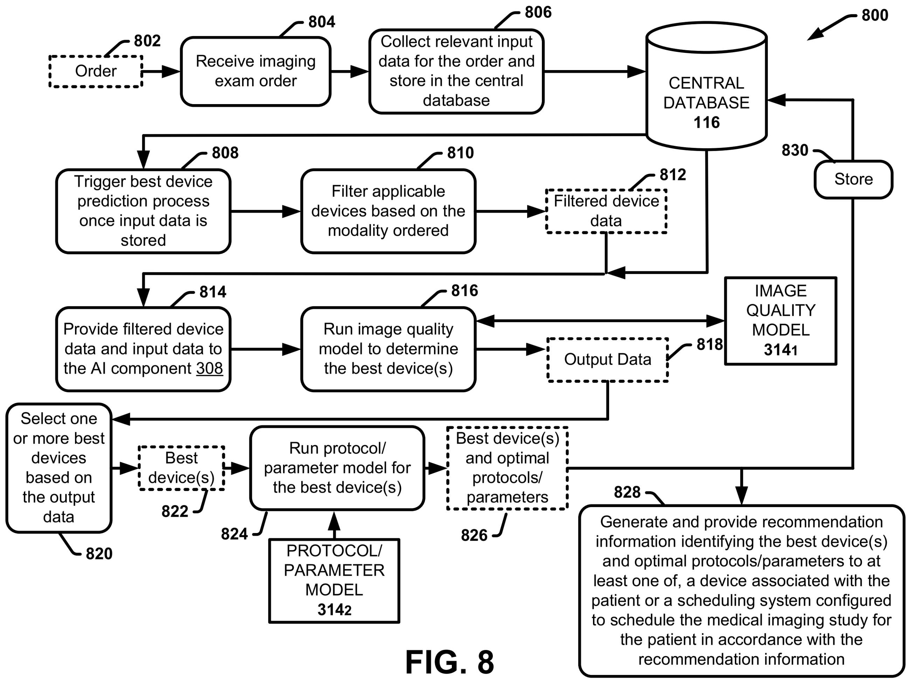

FIG. 8 illustrates another example computer-implemented method 800 for predicting and recommending the best medical imaging device(s) for fulfilling an imaging exam order, in accordance with one or more embodiments described herein. With reference to FIG. 8 in view of FIGs. 1-5, method 800 corresponds to an example method that can be performed by system 100 in accordance with an embodiment.

In accordance with method 800, at 804 the system (e.g., system 100) receives an imaging exam order 802 for a patient to be scheduled and performed. For example, the system can receive the order 802 in response to entering of the order into a RIS connected to the computing device 104 or in another manner, as described above in method 700. The order 802 can comprise information identifying the patient, the modality, a clinical indication for the medical imaging exam, and an anatomical region (i.e., ROI) to be captured in the medical imaging exam. In some implementations, the order may comprise relevant information about the patient, such as information indicating the patient's health status, size, gender, age, and the like. In other implementations, this information can be collected/extracted from the patient's medical records (e.g., in an EMR, EHR, or another source) at 806 based on the patient's ID as included in the order 802.

At 806, the system can collect (e.g., via reception component 302) relevant input data for the order and stores it in the central database 116. For example, the relevant input data can include the order information and the relevant patient information for the patient. The relevant input data also includes the imaging devices information 204, the reference quality information quality information 208, and the reference dose information 210. Additionally, or alternatively, this information may already be included in the central database 116 and/or accessed by the computing device 104 at their corresponding sources.

At 808, the system triggers the best device prediction process once the input data is stored in the central database 116. At 810, the best device prediction process can involve initially filtering the applicable devices (e.g., included in the imaging devices information 204) based on the modality ordered for the exam (e.g., X-ray, CT, nuclear medicine, MRI, and others) to generate filtered device data 812. The filtered device data 812 represents a collection of imaging devices of the same modality, that is the modality ordered for the exam. At 814, the filtered device data 812 and the input data (e.g., the order information and the relevant patient information) are provided to the AI component 308. At 816, the execution component 312 runs an image quality model 314₁ to determine the best device(s). For example, the image quality model 314₁ can correspond to one or more of the ML models 314.

In an embodiment, the image quality model 314₁ can be configured to process input data comprising the order information, the patient parameters and the filtered device data, and generate output data 818 comprising estimated quality measures for the respective filtered devices regarding estimated measures of quality for the exam images capable of being generated by the respective devices. The estimated quality measures can include, for each device, one or more average quality measures and/or one or more highest quality measures. In some implementations, (as applied to X-ray, CT, and nuclear medicine modalities), the output data 818 can also include estimated dose levels expected to be exposed to the patient via the respective imaging devices in association with obtaining the estimated quality measures (e.g., dose for the average quality measures and dose for the highest quality measures).

At 820, the assessment component 316 can determine one or more best devices 822 for the exam based on the output data 818. For example, at 820 the assessment component 316 can select (e.g., via selection component 318) one or more of the imaging devices in the filtered device data 812 based on the results of the image quality model 314₁. For example, the selection component 318 can select the one or more best devices based on the best devices satisfying a quality criterion (e.g., as included in reference quality information 208) and/or a dose criterion (where applicable depending on the modality and as included in reference dose information 210) determined or inferred for the type of the exam and/or the patient.

At 824, the execution component can run a protocol/parameter model 314₂ for the best device(s). For example, the protocol/parameter model 314₂ can correspond to one or more of the ML models 314. The protocol/parameter model 314₂ can be configured to estimate the optimal protocols/parameters (and optionally technician) for the best device(s) 826 that can be used for the exam by the best devices to achieve the quality criterion and/or the dose criterion. For example, as trained on historical data for the best devices regarding historical protocols used, resulting image quality, and corresponding dosages involve, the protocol/parameter model 314₂ can estimate the optimal protocols/parameters for the best devices 822 that can result in satisfying the image quality criterion and as a function of minimizing radiation dose, and/or minimizing imaging scan duration.

At 828, the recommendation component 322 can generate and provide recommendation information (e.g., output data 120') identifying the best device(s) and the optimal protocols/parameters to at least one of, a device associated with the patient or a scheduling system configured to schedule the medical imaging study for the patient in accordance with the recommendation information.

The recommendation information can also be stored in the central database 116 at 830and used by the training component 310 to update (e.g., refine/tune) the image quality model 314₁ and/or the protocol/parameter model 314₂ after the imaging exam has been performed for the patient, as illustrated in FIG. 10. In this regard, the recommendation information can be associated with the order for the patient in the central database 116. After the exam has been performed using the best device (or one of the best devices), information about the details of the exam and the actual results with respect to resulting image quality and whether the quality was diagnostically acceptable, acquisition parameters/protocols used, and radiation dose exposed to the patient can be determined (e.g., via the DICOM data, the image quality component 306, corresponding dose reports associated with exam, the radiology report, and so on) and represent a historical exam entry included in the historical imaging exam information 212. As additional new historical exam entries coupled with recommendation information generated by the order fulfillment component 106 are obtained and aggregated, the training component 310 can update the image quality model 314₁ and/or the protocol/parameter model 314₂ based on differences between the recommendation information and the actual results and using the new historical exam entries as training samples, as described in FIG. 10.

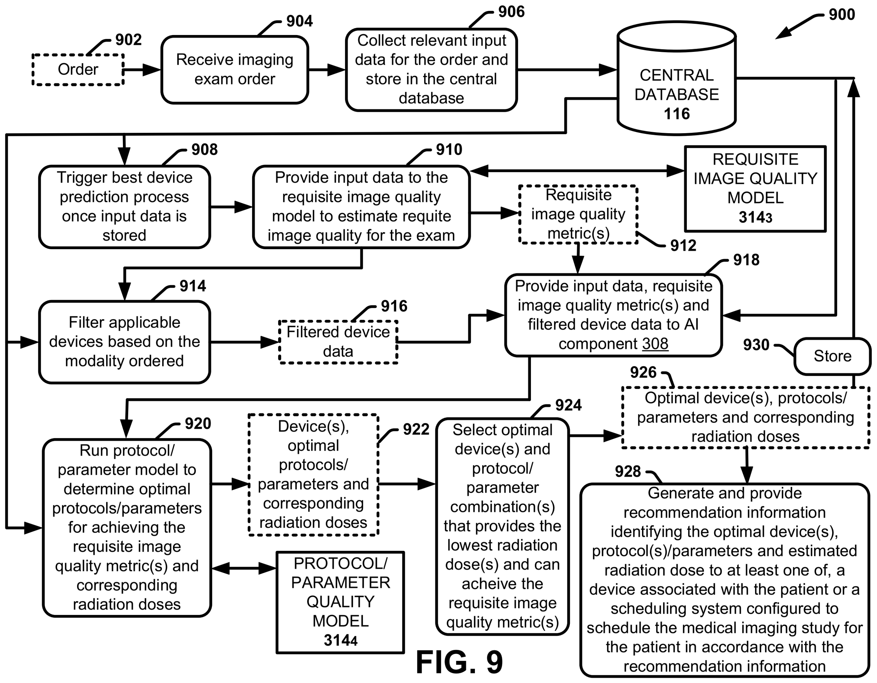

FIG. 9 illustrates another example computer-implemented method 900 for predicting and recommending the best medical imaging device(s) for fulfilling an imaging exam order, in accordance with one or more embodiments described herein. With reference to FIG. 9 in view of FIGs. 1-5, method 900 corresponds to an example method that can be performed by system 100 in accordance with an embodiment.

In accordance with method 900, at 904 the system (e.g., system 100) receives an imaging exam order 902 for a patient to be scheduled and performed. For example, the system can receive the order 902 in response to entering of the order into a RIS connected to the computing device 104 or in another manner, as described above in method 700. The order 902 can comprise information identifying the patient, the modality, a clinical indication for the medical imaging exam, and an anatomical region (i.e., ROI) to be captured in the medical imaging exam. In some implementations, the order may comprise relevant information about the patient, such as information indicating the patient's health status, size, gender, age, and the like. In other implementations, this information can be collected/extracted from the patient's medical records (e.g., in an EMR, EHR, or another source) at 906 based on the patient's ID as included in the order 902.

At 906, the system can collect (e.g., via reception component 302) relevant input data for the order and stores it in the central database 116. For example, the relevant input data can include the order information and the relevant patient information for the patient. The relevant input data also includes the imaging devices information 204, the reference quality information quality information 208, and the reference dose information 210. Additionally, or alternatively, this information may already be included in the central database 116 and/or accessed by the computing device 104 at their corresponding sources.

At 908, the system triggers the best device prediction process once the input data is stored in the central database 116. At 910, the input data is provided as input to a requisite image quality model 314₃ to estimate one or more requisite (e.g., preferred, acceptable, etc.) image quality measures 912 for the exam. For example, the requisite image quality model 314₃ can correspond to one or more of the ML models 314. The requisite image quality metric(s) 912 can reflect a required quality level for the resulting images as tailored based on the exam type (e.g., modality, clinical indication, and ROI) and relevant patient factors (e.g., health status, age, size, gender, etc.). This can include a requisite quality score (representing a combination of different quality metrics) and/or one or more individual quality metrics (e.g., an SNR metric, a CNR metric, a resolution metric, an artifact metric, etc.).

At 914, the filtering component can filter the available devices (e.g., included in the imaging devices information 204) based on the modality ordered for the exam (e.g., X-ray, CT, nuclear medicine, MRI, and others) to generate filtered device data 916. The filtered device data 912 represents a collection of imaging devices of the same modality that may be used for the exam, that is the modality ordered for the exam. At 914, the filtered device data 912 and the requisite image quality metric(s) 912 and the input data (e.g., the order information and the relevant patient information) are provided to the AI component 308.

At 920, the execution component 312 runs a protocol/parameter model 314₄ to determine output data 922 comprising the optimal protocols/parameters that can be employed by the devices identified in the filtered device data 916 to achieve the requisite image quality metric(s) and the corresponding radiation doses (when applicable depending on modality) best device(s). For example, the protocol/parameter model 314₄ can correspond to one or more of the ML models 314. The protocol/parameter model 314₄ can be configured to estimate the optimal protocols/parameters (and optionally technician) for the respective devices that can be used for the to achieve the requisite image quality metric(s) 912. For example, as trained on historical data for the best devices regarding historical protocols used, resulting image quality, and corresponding dosages involve, the protocol/parameter model 314₄ can estimate whether the respective devices can achieve the requisite image quality metric(s) 912 for the type of the exam and the patient profile. For those devices which are capable (e.g., as learned from the historical image quality metrics for same or similar exams and patients), the output information 922 can also identify the optimal protocols/parameters (and corresponding radiation doses expected where applicable) as a function of minimizing radiation dose, and/or minimizing imaging scan duration.

At 924, the selection component 318 can select one or more of the optimal devices and protocol/parameter combinations 926 that provided the lowest radiation doses (and/or scan times) and that can achieve the requisite image quality metric(s). Alternatively, the ranking component 320 can rank the devices from best to least based according to the same. At 928, the recommendation component 322 can generate and provide recommendation information (e.g., output data 120') identifying the best device(s) and the optimal protocols/parameters and the corresponding radiation doses (or the information can be included in the form a ranked device list) to at least one of, a device associated with the patient or a scheduling system configured to schedule the medical imaging study for the patient in accordance with the recommendation information.

The recommendation information can also be stored in the central database 116 at 930 and used by the training component 310 to update (e.g., refine/tune) the requisite image quality model 314₃ and/or the protocol/parameter model 314₄ after the imaging exam has been performed for the patient, as illustrated in FIG. 10. In this regard, the recommendation information can be associated with the order for the patient in the central database 116. After the exam has been performed using the best device (or one of the best devices), information about the details of the exam and the actual results with respect to resulting image quality and whether the quality was diagnostically acceptable, acquisition parameters/protocols used, and radiation dose exposed to the patient can be determined (e.g., via the DICOM data, the image quality component 306, corresponding dose reports associated with exam, the radiology report, and so on) and represent a historical exam entry included in the historical imaging exam information 212. As additional new historical exam entries coupled with recommendation information generated by the order fulfillment component 106 are obtained and aggregated, the training component 310 can update the requisite image quality model 314₃ and/or the protocol/parameter model 314₄ based on differences between the recommendation information and the actual results and using the new historical exam entries as training samples, as described with reference to FIG. 10.

FIG. 10 illustrates an example computer-implemented method 1000 for updating one or more machine learning models to improve the accuracy of predictions related to the best medical imaging device(s) for fulling an exam imaging exam, in accordance with one or more embodiments described herein. With reference to FIG. 10 in view of FIGs. 1-9, in accordance with method 1000, at 1002, the recommendation information generated for received orders for imaging exams is stored in the central database 116 by the order fulfillment evaluation component 106. This recommendation information can include or correspond to output data 120' for the respective orders. For example, for each order, the recommendation information can include but is not limited to, one or more recommended imaging devices for the exam, recommended acquisition protocols, recommended technicians, one or more estimated quality measures, and an estimated dose measure. The recommendation information for each order can be stored with the corresponding order information along with the relevant patient parameters involved (e.g., patient health status, size, age, gender, etc.).

At 1004, the order fulfilment evaluation component 106 receives (e.g., via reception component 302) information describing the imaging exams once performed and stores the information in the historical medical imaging exam information 212 (and/or the central database 116). For example, for each exam, the information received or otherwise collected can include or correspond to the example 214 information shown in FIG. 2. In other words, after an exam for which recommendation information (e.g., corresponding to output data 120' has been generated by the order fulfillment evaluation component 106 has been performed on an imaging device scheduled for the exam, information describing the exam, including the device used, the acquisition protocols/parameters used, the resulting image quality feedback, the dose report and so on, is collected and stored.

At 1006, the information received for the performed exams can be exported to the central database 116 and associated with the corresponding orders and recommendation information. At 1008, the update analysis component 315 can identify target exams performed in accordance with the recommendation information (e.g., using a recommended imaging device, acquisition protocol(s)/parameters(s) and optionally technician) with results (included in the received information at 1004) that differ (e.g., with respect to a defined measure of deviation) from the expected results in terms of expected image quality and/or radiation dose. In some embodiments, this can be based on actual image quality measures determined for the respective exams by the image quality component 306 or another system (e.g., actual measures of SNR, CNR, resolution, artifacts and/or an overall quality score). Additionally, or alternatively, the image quality results associated with the performed exams can include or correspond to a quality score provided by the radiologist (and included in the radiology report) that indicates whether and to what degree the resulting images were diagnostically acceptable or not. With these implementations, those exams for which the results were not diagnostically acceptable can be identified as target exams. In other implementations, those exams for which the resulting quality was lower than estimated yet diagnostically acceptable can also be identified as target exams and used to retrain the requisite image quality model 314₃ as tailored to the different exam type and patient profile combinations represented by the target exams. In other words, at 1008, the update analysis component 315 can perform differential analysis comparing what was recommended and predicted verses what actually happened and resulted from the exam. The results of the differential analysis can be stored in the central database 116 and used for feature learning in association with updating the one or more ML models 314.

At 1010, the training component 310 can generate training data samples 1012 for the target exams. The training data samples 1012 can correspond to training data 402 and/or training data 506. For example, the training data samples 1012 can include, for each target exam, the order information, the relevant patient parameters, optionally the requisite quality measure(s), and the ground truth information which can include or corresponds to actual quality and dose results for the target exam, and in some implementations the acquisition protocol(s)s/parameters(s) used. At 1014, the training component 310 can employ the training data samples 1012 to retrain one or more of the ML models 314 (e.g., image quality model 314₁, protocol/parameter model 314₂, requisite image quality model 314₃, image quality model 314₁, and the like) in accordance with training process 500 and/or the model building phase 510 of training process 500, resulting in improved versions of the ML models 314. At 1016, the AI component 308 can store (e.g., in memory 110, central database 116 and/or another suitable memory device accessibly to the computing device 104) and employ the updated versions of the ML models 314 for future predictions.

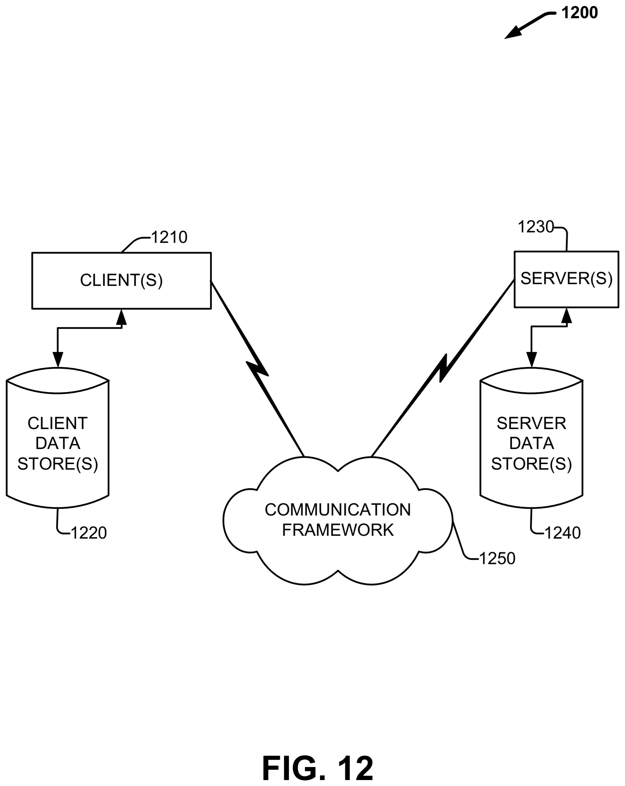

In order to provide a context for the various aspects of the disclosed subject matter, FIGS. 11 and 12 as well as the following discussion are intended to provide a brief, general description of a suitable environment in which the various aspects of the disclosed subject matter may be implemented.

With reference to FIG. 11, a suitable environment 1100 for implementing various aspects of this disclosure includes a computer 1112. The computer 1112 includes a processing unit 1114, a system memory 1116, and a system bus 1118. The system bus 1118 couples system components including, but not limited to, the system memory 1116 to the processing unit 1114. The processing unit 1114 can be any of various available processors. Dual microprocessors and other multiprocessor architectures also can be employed as the processing unit 1114.

The system bus 1118 can be any of several types of bus structure(s) including the memory bus or memory controller, a peripheral bus or external bus, and/or a local bus using any variety of available bus architectures including, but not limited to, Industrial Standard Architecture (ISA), Micro-Channel Architecture (MSA), Extended ISA (EISA), Intelligent Drive Electronics (IDE), VESA Local Bus (VLB), Peripheral Component Interconnect (PCI), Card Bus, Universal Serial Bus (USB), Advanced Graphics Port (AGP), Personal Computer Memory Card International Association bus (PCMCIA), Firewire (IEEE 1394), and Small Computer Systems Interface (SCSI).

The system memory 1116 includes volatile memory 1120 and nonvolatile memory 1122. The basic input/output system (BIOS), containing the basic routines to transfer information between elements within the computer 1112, such as during start-up, is stored in nonvolatile memory 1122. By way of illustration, and not limitation, nonvolatile memory 1122 can include read only memory (ROM), programmable ROM (PROM), electrically programmable ROM (EPROM), electrically erasable programmable ROM (EEPROM), flash memory, or nonvolatile random access memory (RAM) (e.g., ferroelectric RAM (FeRAM). Volatile memory 1120 includes random access memory (RAM), which acts as external cache memory. By way of illustration and not limitation, RAM is available in many forms such as static RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM.

Computer 1112 also includes removable/non-removable, volatile/nonvolatile computer storage media. FIG. 11 illustrates, for example, a disk storage 1124. Disk storage 1124 includes, but is not limited to, devices like a magnetic disk drive, floppy disk drive, tape drive, Jaz drive, Zip drive, LS-110 drive, flash memory card, or memory stick. The disk storage 1124 also can include storage media separately or in combination with other storage media including, but not limited to, an optical disk drive such as a compact disk ROM device (CD-ROM), CD recordable drive (CD-R Drive), CD rewritable drive (CD-RW Drive) or a digital versatile disk ROM drive (DVD-ROM). To facilitate connection of the disk storage devices 1124 to the system bus 1118, a removable or non-removable interface is typically used, such as interface 1126.

FIG. 11 also depicts software that acts as an intermediary between users and the basic computer resources described in the suitable operating environment 1100. Such software includes, for example, an operating system 1128. Operating system 1128, which can be stored on disk storage 1124, acts to control and allocate resources of the computer system 1112. System applications 1130 take advantage of the management of resources by operating system 1128 through program modules 1132 and program data 1134, e.g., stored either in system memory 1116 or on disk storage 1124. It is to be appreciated that this disclosure can be implemented with various operating systems or combinations of operating systems.

A user enters commands or information into the computer 1112 through input device(s) 1136. Input devices 1136 include, but are not limited to, a pointing device such as a mouse, trackball, stylus, touch pad, keyboard, microphone, joystick, game pad, satellite dish, scanner, TV tuner card, digital camera, digital video camera, web camera, and the like. These and other input devices connect to the processing unit 1114 through the system bus 1118 via interface port(s) 1138. Interface port(s) 1138 include, for example, a serial port, a parallel port, a game port, and a universal serial bus (USB). Output device(s) 1140 use some of the same type of ports as input device(s) 1136. Thus, for example, a USB port may be used to provide input to computer 1112, and to output information from computer 1112 to an output device 1140. Output adapter 1142 is provided to illustrate that there are some output devices 1140 like monitors, speakers, and printers, among other output devices 1140, which require special adapters. The output adapters 1142 include, by way of illustration and not limitation, video and sound cards that provide a means of connection between the output device 1140 and the system bus 1118. It should be noted that other devices and/or systems of devices provide both input and output capabilities such as remote computer(s) 1144.

Computer 1112 can operate in a networked environment using logical connections to one or more remote computers, such as remote computer(s) 1144. The remote computer(s) 1144 can be a personal computer, a server, a router, a network PC, a workstation, a microprocessor based appliance, a peer device or other common network node and the like, and typically includes many or all of the elements described relative to computer 1112. For purposes of brevity, only a memory storage device 1146 is illustrated with remote computer(s) 1144. Remote computer(s) 1144 is logically connected to computer 1112 through a network interface 1148 and then physically connected via communication connection 1150. Network interface 1148 encompasses wire and/or wireless communication networks such as local-area networks (LAN), wide-area networks (WAN), cellular networks, etc. LAN technologies include Fiber Distributed Data Interface (FDDI), Copper Distributed Data Interface (CDDI), Ethernet, Token Ring and the like. WAN technologies include, but are not limited to, point-to-point links, circuit switching networks like Integrated Services Digital Networks (ISDN) and variations thereon, packet switching networks, and Digital Subscriber Lines (DSL).

Communication connection(s) 1150 refers to the hardware/software employed to connect the network interface 1148 to the bus 1118. While communication connection 1150 is shown for illustrative clarity inside computer 1112, it can also be external to computer 1112. The hardware/software necessary for connection to the network interface 1148 includes, for exemplary purposes only, internal and external technologies such as, modems including regular telephone grade modems, cable modems and DSL modems, ISDN adapters, and Ethernet cards.

FIG. 12 is a schematic block diagram of a sample-computing environment 1200 with which the subject matter of this disclosure can interact. The system 1200 includes one or more client(s) 1210. The client(s) 1210 can be hardware and/or software (e.g., threads, processes, computing devices). The system 1200 also includes one or more server(s) 1230. Thus, system 1200 can correspond to a two-tier client server model or a multi-tier model (e.g., client, middle tier server, data server), amongst other models. The server(s) 1230 can also be hardware and/or software (e.g., threads, processes, computing devices). The servers 1230 can house threads to perform transformations by employing this disclosure, for example. One possible communication between a client 1210 and a server 1230 may be in the form of a data packet transmitted between two or more computer processes.

The system 1200 includes a communication framework 1250 that can be employed to facilitate communications between the client(s) 1210 and the server(s) 1230. The client(s) 1210 are operatively connected to one or more client data store(s) 1220 that can be employed to store information local to the client(s) 1210. Similarly, the server(s) 1230 are operatively connected to one or more server data store(s) 1240 that can be employed to store information local to the servers 1230.

It is to be noted that aspects or features of this disclosure can be exploited in substantially any wireless telecommunication or radio technology, e.g., Wi-Fi; Bluetooth; Worldwide Interoperability for Microwave Access (WiMAX); Enhanced General Packet Radio Service (Enhanced GPRS); Third Generation Partnership Project (3GPP) Long Term Evolution (LTE); Third Generation Partnership Project 2 (3GPP2) Ultra Mobile Broadband (UMB); 3GPP Universal Mobile Telecommunication System (UMTS); High Speed Packet Access (HSPA); High Speed Downlink Packet Access (HSDPA); High Speed Uplink Packet Access (HSUPA); GSM (Global System for Mobile Communications) EDGE (Enhanced Data Rates for GSM Evolution) Radio Access Network (GERAN); UMTS Terrestrial Radio Access Network (UTRAN); LTE Advanced (LTE-A); etc. Additionally, some or all of the aspects described herein can be exploited in legacy telecommunication technologies, e.g., GSM. In addition, mobile as well non-mobile networks (e.g., the Internet, data service network such as internet protocol television (IPTV), etc.) can exploit aspects or features described herein.

While the subject matter has been described above in the general context of computer-executable instructions of a computer program that runs on a computer and/or computers, those skilled in the art will recognize that this disclosure also can or may be implemented in combination with other program modules. Generally, program modules include routines, programs, components, data structures, etc. that perform particular tasks and/or implement particular abstract data types. Moreover, those skilled in the art will appreciate that the inventive methods may be practiced with other computer system configurations, including single-processor or multiprocessor computer systems, mini-computing devices, mainframe computers, as well as personal computers, hand-held computing devices (e.g., PDA, phone), microprocessor-based or programmable consumer or industrial electronics, and the like. The illustrated aspects may also be practiced in distributed computing environments where tasks are performed by remote processing devices that are linked through a communications network. However, some, if not all aspects of this disclosure can be practiced on stand-alone computers. In a distributed computing environment, program modules may be located in both local and remote memory storage devices.

As used in this application, the terms "component," "system," "platform," "interface," and the like, can refer to and/or can include a computer-related entity or an entity related to an operational machine with one or more specific functionalities. The entities disclosed herein can be either hardware, a combination of hardware and software, software, or software in execution. For example, a component may be, but is not limited to being, a process running on a processor, a processor, an object, an executable, a thread of execution, a program, and/or a computer. By way of illustration, both an application running on a server and the server can be a component. One or more components may reside within a process and/or thread of execution and a component may be localized on one computer and/or distributed between two or more computers.

In another example, respective components can execute from various computer readable media having various data structures stored thereon. The components may communicate via local and/or remote processes such as in accordance with a signal having one or more data packets (e.g., data from one component interacting with another component in a local system, distributed system, and/or across a network such as the Internet with other systems via the signal). As another example, a component can be an apparatus with specific functionality provided by mechanical parts operated by electric or electronic circuitry, which is operated by a software or firmware application executed by a processor. In such a case, the processor can be internal or external to the apparatus and can execute at least a part of the software or firmware application. As yet another example, a component can be an apparatus that provides specific functionality through electronic components without mechanical parts, wherein the electronic components can include a processor or other means to execute software or firmware that confers at least in part the functionality of the electronic components. In an aspect, a component can emulate an electronic component via a virtual machine, e.g., within a cloud computing system.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in the subject specification and annexed drawings should generally be construed to mean "one or more" unless specified otherwise or clear from context to be directed to a singular form.

As used herein, the terms "example" and/or "exemplary" are utilized to mean serving as an example, instance, or illustration. For the avoidance of doubt, the subject matter disclosed herein is not limited by such examples. In addition, any aspect or design described herein as an "example" and/or "exemplary" is not necessarily to be construed as preferred or advantageous over other aspects or designs, nor is it meant to preclude equivalent exemplary structures and techniques known to those of ordinary skill in the art.

Various aspects or features described herein can be implemented as a method, apparatus, system, or article of manufacture using standard programming or engineering techniques. In addition, various aspects or features disclosed in this disclosure can be realized through program modules that implement at least one or more of the methods disclosed herein, the program modules being stored in a memory and executed by at least a processor. Other combinations of hardware and software or hardware and firmware can enable or implement aspects described herein, including a disclosed method(s). The term "article of manufacture" as used herein can encompass a computer program accessible from any computer-readable device, carrier, or storage media. For example, computer readable storage media can include but are not limited to magnetic storage devices (e.g., hard disk, floppy disk, magnetic strips...), optical discs (e.g., compact disc (CD), digital versatile disc (DVD), blu-ray disc (BD) ...), smart cards, and flash memory devices (e.g., card, stick, key drive...), or the like.

As it is employed in the subject specification, the term "processor" can refer to substantially any computing processing unit or device comprising, but not limited to, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. Further, processors can exploit nano-scale architectures such as, but not limited to, molecular and quantum-dot based transistors, switches and gates, in order to optimize space usage or enhance performance of user equipment. A processor may also be implemented as a combination of computing processing units.

In this disclosure, terms such as "store," "storage," "data store," data storage," "database," and substantially any other information storage component relevant to operation and functionality of a component are utilized to refer to "memory components," entities embodied in a "memory," or components comprising a memory. It is to be appreciated that memory and/or memory components described herein can be either volatile memory or nonvolatile memory, or can include both volatile and nonvolatile memory.

By way of illustration, and not limitation, nonvolatile memory can include read only memory (ROM), programmable ROM (PROM), electrically programmable ROM (EPROM), electrically erasable ROM (EEPROM), flash memory, or nonvolatile random access memory (RAM) (e.g., ferroelectric RAM (FeRAM). Volatile memory can include RAM, which can act as external cache memory, for example. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), direct Rambus RAM (DRRAM), direct Rambus dynamic RAM (DRDRAM), and Rambus dynamic RAM (RDRAM). Additionally, the disclosed memory components of systems or methods herein are intended to include, without being limited to including, these and any other suitable types of memory.

It is to be appreciated and understood that components, as described with regard to a particular system or method, can include the same or similar functionality as respective components (e.g., respectively named components or similarly named components) as described with regard to other systems or methods disclosed herein.

What has been described above includes examples of systems and methods that provide advantages of this disclosure. It is, of course, not possible to describe every conceivable combination of components or methods for purposes of describing this disclosure, but one of ordinary skill in the art may recognize that many further combinations and permutations of this disclosure are possible. Furthermore, to the extent that the terms "includes," "has," "possesses," and the like are used in the detailed description, claims, appendices and drawings such terms are intended to be inclusive in a manner similar to the term "comprising" as "comprising" is interpreted when employed as a transitional word in a claim.

## Claims

1. A method, comprising:
receiving, by a system comprising at least one processor, an order requesting performance of a medical imaging exam on a patient, the order comprising order information identifying a modality of the medical imaging exam, a clinical indication for the medical imaging exam, and an anatomical region to be captured in the medical imaging exam;
identifying, by the system, different medical imaging devices capable of performing the medical imaging exam based on the modality;
employing, by the system, one or more first machine learning processes to estimate measures of quality of the medical imaging exam capable of being generated by the different medical imaging devices based on the order information and historical imaging exam information regarding historical medical imaging exams performed by the different medical imaging devices that satisfy the order information;
selecting, by the system, a subset of the different medical imaging devices based on respective measures of quality for the subset satisfying a quality criterion;
generating, by the system, recommendation information identifying the subset; and
providing, by the system, the recommendation information to at least one of, a device associated with the patient or a scheduling system configured to schedule the medical imaging exam for the patient in accordance with the recommendation information.

2. The method of claim 1, wherein the employing comprises:
training, by the system, one or more machine learning models to estimate training measures of quality corresponding to the measures of quality based on the historical imaging exam information, resulting in one or more trained versions of the one or more machine learning models; and
applying, by the system, the order information and device information identifying the different medical imaging devices as input to the one or more trained versions to generate the measures of quality.

3. The method of claim 1, wherein the quality criterion comprises an acceptable measure of quality for the medical imaging exam.

4. The method of claim 3, further comprising:
employing, by the system, the one or more first machine learning processes or one or more second machine learning processes to estimate the acceptable measure of quality for the medical imaging exam based on the order information and the historical imaging exam information.

5. The method of claim 3, further comprising:
employing, by the system, the one or more first machine learning processes or one or more second machine learning processes to estimate, based on the order information and the historical imaging exam information, radiation doses capable of being exposed to the patient via the different medical imaging devices in association with performing the medical imaging exam and achieving the acceptable measure of quality, wherein the selecting the subset is further based on respective radiation doses for the subset satisfying a radiation dose criterion.

6. The method of claim 5, wherein the employing comprises:
training, by the system, one or more machine learning models to estimate training radiation doses corresponding to the radiation doses based on the historical imaging exam information, resulting in one or more trained versions of the one or more machine learning models; and
applying, by the system, the order information, device information identifying the different medical imaging devices, and the acceptable measure of quality as input to the one or more trained versions to generate the radiation doses.

7. The method of claim 5, wherein the employing the one or more first machine learning processes or the one or more second machine learning processes further comprises determining respective acquisition protocols capable of being used by the subset in association with performing the medical imaging exam and achieving the acceptable measure of quality with the respective radiation doses for the subset that satisfy the radiation dose criterion, and wherein the generating the recommendation information further comprises including acquisition protocol information with the recommendation information identifying the respective acquisition protocols for the subset.

8. The method of claim 7, wherein the selecting the subset is further based on the respective acquisition protocols for the subset satisfying an acquisition protocol criterion.

9. The method of claim 5, wherein the employing the one or more first machine learning processes or the one or more second machine learning processes further comprises determining respective acquisition parameters capable of being used by the subset in association with performing the medical imaging exam and achieving the acceptable measure of quality with the respective radiation doses for the subset that satisfy the radiation dose criterion, and wherein the generating the recommendation information further comprises including acquisition parameter information with the recommendation information identifying the respective acquisition parameters for the subset.

10. The method of claim 9, wherein the selecting the subset is further based on the respective acquisition parameters for the subset satisfying an acquisition parameter criterion.

11. The method of claim 5, wherein the employing the one or more first machine learning processes or the one or more second machine learning processes further comprises determining respective technicians capable of being used by the subset in association with performing the medical imaging exam and achieving the acceptable measure of quality with the respective radiation doses for the subset that satisfy the radiation dose criterion, and wherein the generating the recommendation information further comprises including technician information with the recommendation information identifying the respective technicians for the subset.

12. The method of claim 1, further comprising:
determining, by the system, one or more patient parameters relevant to the medical imaging exam included in an electronic medical record for the patient, wherein the employing further comprises employing the one or more first machine learning processes to estimate the measures of quality based on the one or more patient parameters.

13. A system, comprising:
at least one memory that stores computer-executable components; and
at least one processor that executes the computer-executable components stored in the at least one memory, wherein the computer-executable components comprise:
a reception component that receives an order requesting performance of a medical imaging exam on a patient, the order comprising order information identifying a modality of the medical imaging exam, a clinical indication for the medical imaging exam, and an anatomical region to be captured in the medical imaging exam;
a filtering component that identifies different medical imaging devices capable of performing the medical imaging exam based on the modality;
an artificial intelligence component that employs more first machine learning processes to estimate measures of quality of the medical imaging exam capable of being generated by the different medical imaging devices based on the order information and historical imaging exam information regarding historical medical imaging exams performed by the different medical imaging devices that satisfy the order information;
an assessment component that selects a subset of the different medical imaging devices based on respective measures of quality for the subset satisfying a quality criterion;
a recommendation component that generates recommendation information identifying the subset and provides the recommendation information to at least one of, a device associated with the patient or a scheduling system configured to schedule the medical imaging exam for the patient in accordance with the recommendation information.

14. The system of claim 13, wherein the computer-executable components further comprise:
a training component that trains one or more machine learning models to estimate training measures of quality corresponding to the measures of quality based on the historical imaging exam information, resulting in one or more trained versions of the one or more machine learning models; and
an execution component that applies the order information and device information identifying the different medical imaging devices as input to the one or more trained versions to generate the measures of quality.

15. A non-transitory machine-readable storage medium, comprising executable instructions that, when executed by a processor, facilitate performance of operations, comprising:
receiving an order requesting performance of a medical imaging exam on a patient, the order comprising order information identifying a modality of the medical imaging exam, a clinical indication for the medical imaging exam, and an anatomical region to be captured in the medical imaging exam;
identifying different medical imaging devices capable of performing the medical imaging exam based on the modality;
employing one or more first machine learning processes to estimate measures of quality of the medical imaging exam capable of being generated by the different medical imaging devices based on the order information and historical imaging exam information regarding historical medical imaging exams performed by the different medical imaging devices that satisfy the order information;
selecting a subset of the different medical imaging devices based on respective measures of quality for the subset satisfying a quality criterion;
generating recommendation information identifying the subset; and
providing the recommendation information to at least one of, a device associated with the patient or a scheduling system configured to schedule the medical imaging exam for the patient in accordance with the recommendation information.
